# EUROPEAN PATENT APPLICATION

(11) **EP 3 943 595 A1**
(43) Date of publication of application: **26.01.2022**
(21) Application number: 20930653.9
(22) Date of filing: 18.12.2020
(51) Int. Cl.: C12N 1/21, C12P 13/08, C12N 15/53, C12R 1/19

(54) **RECOMBINANT MICROORGANISM FOR PRODUCING L-VALINE, CONSTRUCTION METHOD THEREFOR AND USE THEREOF**

(30) Priority: 27.05.2020 CN 202010460035
(71) Applicant: Anhui Huaheng Biotechnology Co., Ltd., Suangfeng Industrial Park Hefei, Anhui 21131 (CN); Tianjin Institute Of Industrial Biotechnology, Chinese Academy of Sciences, Tianjin 300308 (CN); BAYANNUR HUAHENG BIOTECHNOLOGY CO., LTD, Shanba Town, Hangjinhou Banner Bayannaoer, Inner Mongolia 015000 (CN)
(72) Inventor: ZHANG, Xueli, Tianjin 300308 (CN); GUO, Henghua, Hefei, Anhui 21131 (CN); LIU, Pingping, Tianjin 300308 (CN); ZHANG, Dongzhu, Hefei, Anhui 21131 (CN); TANG, Jinlei, Tianjin 300308 (CN); HAN, Chengxiu, Bayannaoer, Inner Mongolia 015000 (CN); TANG, Siqing, Bayannaoer, Inner Mongolia 015000 (CN); LIU, Shupeng, Hefei, Anhui 21131 (CN); MA, Yanhe, Tianjin 300308 (CN)
(74) Representative: Biggi, Cristina
(86) International application number: PCT/CN2020/137779
(87) International publication number: WO 2021/238184

(57) **Abstract**

Related are a recombinant microorganism for producing L-valine, a construction method and an application thereof. Through enhancing amino acid dehydrogenase activity of L-valine fermentation strain, and/or activating an Entner-Doudoroff (ED) metabolic pathway, a problem in L-valine fermentation process that reducing power is unbalanced is solved, thereby the titer and yield of L-valine produced by *Escherichia coli* are improved, and L-valine was produced by one-step anaerobic fermentation.

## Description

### Technical Field

The disclosure relates to a construction method of a recombinant microorganism for producing L-valine, the recombinant microorganism obtained by the construction method, specifically recombinant *Escherichia coli,* and a method for producing L-valine through a fermentation method.

### Background

L-valine, as one of three branched-chain amino acids (BCAA), is an essential amino acid. It may not be synthesized in humans and animals, and can only be acquired by supplementation in vitro. Nowadays, L-valine has been widely used in the fields of food and medicine, mainly including food additive, nutritional supplement and flavoring agent and the like; and widely used in preparation of cosmetics, as precursors of antibiotics or herbicides and the like. Additionally, along with the increasing demands for feed quality and ratio, the role of L-valine in the feed additive industry will become more and more important in the future. And this will result the expand of its demand and great potential in the future market.

L-valine can be directly synthesized by microbial cells. However, the production capacity of wild-type cells is greatly limited by the intracellular regulatory network, such as feedback inhibition and so on. To obtain a strain capable of efficiently producing L-valine by fermentation process, these self-regulatory mechanisms of the microbial cells must be effectively eliminated. At present,

L-valine is mainly produced through fermentation method in the world. Strains used for fermentative production of L-valine are mostly obtained by mutagenesis, and original strains mainly include *Corynebacterium glutamicum, Brevibacterium flavum* and the like. For example, starting from a *Brevibacterium flavum,* Ning Chen et al. produced mutant strains by using protoplast ultraviolet mutagenesis in combination with the DES chemical mutagenesis. Finally, an efficiently L-valine-producing strain TV2564 was screened and selected, and its L-valine titer was as high as 29.39 g/L. But, a notable fact is that strains obtained by traditional mutagenesis have many limites, such as strong randomness, unclear genetic background, producing by-products during fermentation, and not easy to modified for obtaining much more highly efficient strains.

With the rapid development of synthetic biology and metabolic engineering in recent years, recombinant engineered strains have been developed and achieved good results.These strains can efficiently produce L-valine, have clear genetic background, and are easy to be cultivated. Xixian Xie et al. significantly improved L-valine production level of a starting strain VHY03 in the flask fermentation by integrating alsS gene encoding acetolactate synthase of Bacillus substilis, relieving the feedback inhibition of L-valine to key enzymes involved in the synthetic pathway, and integrating the mutant spoTM gene encoding the ppGpp3'-pyrophosphohydrolase of Escherichia coli to enhance the supply of pyruvate. In 2007, Sang Yup Lee's group developed an L-valine-producing strain starting from the Escherichia coli w3110 by in combination of different strategies, such as the rational metabolic engineering, transcriptome analysis and genetic modification, and gene knockout. This strain can produce L-valine in batch culture under aerobic conditions. But it needs continuous oxygen supply during the culture process and L-isoleucine needs to be added to ensure the normal growth. Furthermore, this research group reconstructed a new L-valine producing strain using a similar modification strategies from Escherichia coli strain W which has higher tolerance to L-valine. This strain also needs to be cultured under aerobic condition for L-valine production.

Although the titer of L-valine has been improved, the production process of L-valine all need to be carried out under aerobic or microaerobic conditions and unable by a one-step anaerobic fermentation process in above reports. The aerobic process requires air during the production process and consumes a lot of energy. More critically, a considerable part of carbon sources enters a tricarboxylic acid cycle (TCA) and is consumed by cell growth resulting that the yield of L-valine is always much lower than the theoretical maximum. Therefore, the aerobic process always has high cost, requirements for devices, and complicated operation steps. Compared with aerobic process, anaerobic process has the advantages of low energy consumption and high conversion rate. There is no need to pass air in the production process, which greatly saves energy consumption. The product conversion rate is usually close to the theoretical maximum. Anaerobic fermentation for the production of amino acids was first realized in the production of alanine. At present, there is no report on pure anaerobic fermentation technology for the production of other amino acids.

In addition, the overexpression of key genes involved in the metabolic engineering of L-valine strains are always plasmid-borne, so that antibiotics needs to be added in the fermentation process to maintain the existence of the plasmid. As a result, the production cost is greatly increased and a risk of plasmid loss exists in industrial production. Therefore, there is still a need in the field to provide high-yield, energy-saving, simple and stable recombinant microorganisms for producing L-valine and a corresponding production and preparation method for L-valine.

### Summary

The disclosure is capable of, through enhancing amino acid dehydrogenase activity of an L-valine fermentation strain and/or activating an Entner-Doudoroff (ED) pathway, solving a problem in L-valine fermentation process that reducing power is unbalanced, thereby improving the titer and yield of L-valine generated by *Escherichia coli,* and realizing fermentation-valine production by one-step anaerobic fermentation.

The first aspect of the disclosure is to provide a construction method for construction of a recombinant microorganism producing L-valine. The recombinant microorganism obtained by this method has stable genetic background and balanced L-valine reducing power, and is suitable for one-step anaerobic fermentation.

In the present disclosure, "enhancement" of enzyme activity refers to enhancement of intracellular activity of one or more enzymes which has corresponding gene in the microorganism. The enhancement of the activity may be achieved by any suitable methods known in the field, for example, by overexpression, it includes but not limited to increasing the copy number of the gene or allele, modifying a nucleotide sequence for guiding or controlling gene expression, using a strong promoter to increase protein activity or concentration by 10%-500% compared to an initial microbial level.

In the present disclosure, "activation" refers to generation or enhancement of a metabolic pathway to be activated by a mode known in the field, such as related genes on the metabolic pathway are transferred into a microorganism, so that the metabolic pathway is generated; or the related genes of the metabolic pathway to be activated are placed under the control of an appropriate regulatory element, for example, placed under an enhancer sequence, so that the expression intensity of these genes is improved.

In one embodiment, an amino acid dehydrogenase gene is transferred into the microorganism, so that the enzyme activity is enhanced.

In one preferred embodiment, through transferring the amino acid dehydrogenase gene into the microorganism, the enzyme activity is enhanced.

In one embodiment, a step of activating an ED pathway in the microorganisms is included.

In some preferred embodiments, steps of transferring the amino acid dehydrogenase gene into the microorganism and activating the ED pathway in the microorganism are included.

In one embodiment, the amino acid dehydrogenase gene transferred in the disclosure is exogenous to the microorganism transferred. The amino acid dehydrogenase gene may be a corresponding gene from any microorganisms such as *Lactococcus,* and *Bacillus.*

In one embodiment, the amino acid dehydrogenase gene is NADH-dependent.

During anaerobic fermentation, glucose metabolism is mainly through the glycolysis pathway (EMP pathway). While 1 mol of glucose is metabolized to generate 2 mol of pyruvate, 2 mol of ATP and 2 mol of NADH may be generated. Therefore, this causes a problem of unbalanced supply of redox power in metabolically engineered *Escherichia coli* under anaerobic conditions, namely, the NADH is excessive, and supply of NADPH is insufficient. Therefore, in order to efficiently produce L-valine under anaerobic conditions, the problem of cofactor imbalance must be solved. The selection of the NADH-dependent amino acid dehydrogenase gene in the disclosure may cause the excessive NADH to be consumed under anaerobic conditions and solve the problem of reducing power imbalance during the anaerobic fermentation.

In one embodiment, the amino acid dehydrogenase gene is a leucine dehydrogenase gene, and preferably, the leucine dehydrogenase gene is leuDH.

In one embodiment, the activation of the ED pathway includes steps of improving the expression intensity of a 6-phosphate glucose dehydrogenase *(zwf)* gene, a lactonase encoding gene *(pgl),* a 6-phosphogluconate dehydratase gene *(edd),* and a 2-ketone-3-deoxy-6-phosphogluconate aldolase gene *(eda).*

It may be understood by those skilled in the art that the expression intensity of the genes may be improved by increasing copy numbers of the *zwf, pgl, edd* and *eda* genes, or by placing these genes under the control of the appropriate regulatory elements.

In one embodiment, a step of transferring an acetohydroxy acid reductoisomerase encoding gene into the microorganism is further included, so that the enzyme activity is enhanced; and preferably, the acetohydroxy acid reductoisomerase encoding gene is selected from *ilvC.*

The "transfer" may exist in the microorganism in any suitable forms known in the field, for example, in the form of a plasmid or the form of being integrated into a genome. In one embodiment, the enzyme encoding gene integrated into the genome is placed under the control of a suitable regulatory element.

The regulatory element is selected from an M1-93 artificial regulatory element, an MRS1 artificial regulatory element, a RBS artificial regulatory element or an M1-46 artificial regulatory element.

In one embodiment, the M1-93 artificial regulatory element regulates a *leuDH* gene, a *zwf* gene and a *pgl* gene*.*

In one embodiment, the MRS1 artificial regulatory element regulates an *edd* gene.

In one embodiment, the RBS artificial regulatory element regulates a gene *eda.*

In one embodiment, the M1-46 artificial regulatory element regulates an *ilvC* gene*.*

In one embodiment, the disclosure further includes a step of knocking out a 6-phosphoglucokinase gene *(pfkA)* of the above recombinant microorganism.

In one embodiment, the disclosure further includes the following modifications to one or more of the following enzyme genes of the above recombinant microorganism, so that the activity of these enzymes is reduced or inactivated.
(1) Knocking out a methylglyoxal synthase *(mgsA)* gene;
(2) knocking out a lactate dehydrogenase *(IdhA)* gene;
(3) knocking out phosphoacetyl transferase (pta) and/or acetate kinase *(ackA)* genes;
(4) knocking out propionate kinase *(tdcD)* and/or formate acetyltransferase *(tdcE)* genes;
(5) knocking out an alcohol dehydrogenase *(adhE)* gene; and
(6) knocking out fumarate reductase *(frd)* and/or pyruvate formate lyase *(pflB)* genes.

The "knockout" mentioned in the present disclosure refers to gene knockout using modes known in the field, so that the activity of the enzyme is reduced or inactivated. The knockout operation is aimed at an endogenous enzyme gene of the original microorganism, so that the above endogenous enzyme activity of the microorganism is reduced or inactivated.

An encoding sequence of the enzyme gene in the above (1)-(6) may also be substituted with an encoding sequence of another gene by means of genetic engineering such as homologous recombination, thereby the above endogenous enzyme activity of the microorganism is reduced or inactivated. The gene to replace these endogenous enzymes may be a gene to be enhanced for expression, such as the above *ilvC* gene or *leuDH* gene.

In one embodiment, it further includes:
(7) enhancing activity of acetolactate synthase (AHAS) and/or dihydroxy acid dehydratase *(ilvD)* in the recombinant microorganism of the disclosure.

In one preferred embodiment, the AHAS is selected from *ilvBN, ilvGM* or *ilvlH,* and the activity of at least one of them is enhanced.

In one preferred embodiment, the *ilvBN* and *ilvGM* genes may be placed under the control of the appropriate regulatory element, so that the expressions of the genes are enhanced. The regulatory element is preferably the M1-93 artificial regulatory element.

In one preferred embodiment, the activity of *ilvlH* is enhanced by releasing the feedback inhibition of valine to the *ilvlH,* for example, the feedback inhibition of valine to the *ilvlH* is released by mutating the *ilvH* gene.

For the involved acetolactate synthase used in biologically synthesizing L-valine, in addition to an isozyme II (herein also referred to as AHAS II), an isozyme III (herein also referred to as AHAS III) is also known. The AHASIII is encoded by an ilvlH operon, and the operon is formed by ilvl encoding a large subunit (catalytic subunit) and ilvH encoding a small subunit (control subunit). The AHAS III is feedback inhibited by L-valine. A reported method may be used to mutate the ilvl gene, such as amino acid substitution of ilvH 14 Gly→Asp (Vyazmensky, M. et al., "Biochemistry" 35:10339-10346 (1996)) and/or ilvH 17Ser→Phe (US6737255B2); and ilvH612 (De Felice et al., "Journal of Bacteriology" 120:1058-1067 (1974)) and the like.

In one embodiment, activity of a dihydroxy acid dehydratase (*ilvD*) in the recombinant microorganism of the disclosure is enhanced, for example, the *ilvD* gene is transferred into the microorganism to enhance the activity of the *ilvD.*

An operation in the item (7) is performed optionally in combination with any one or more of the above modifications (1)-(6).

In one embodiment, the operation is performed in combination with the modification of the item (2).

In one embodiment, the operation is performed in combination with the modification of the item (6).

In one embodiment, the operation is performed in combination with the modifications of the item (2) and the item (5).

In one embodiment, the operation is performed in combination with the modifications of the item (2) and the item (6).

In one embodiment, the operation is performed in combination with the modifications of the items (1) and (3)-(6).

In one embodiment, the operation is performed in combination with the modifications of the items (1)-(6).

In one embodiment, optionally, the knockout of the item (1) is achieved by substituting the endogenous *mgsA* gene of the microorganism with the *ilvC* gene.

In one embodiment, the knockout of the item (6) is achieved by substituting the endogenous *pflB* gene of the microorganism with the *ilvD* gene, and/or substituting the endogenous *frd* gene of the microorganism with the leuDH gene.

The substitution may be that, in a mode known to those skilled in the art, an encoding sequence of a gene to be inserted is integrated into a substituted gene in the microbial chromosome, so that the gene encoding sequence in the original site is substituted with the encoding sequence of the integrated inserted gene.

Preferably, the replacements of the *ilvC, ilvD* and *leuDH* occur simultaneously.

In one embodiment, the microorganism is *Escherichia coli.*

In one embodiment, the microorganism is *Escherichia coli ATCC* 8739.

In one embodiment, at least one regulatory element is used to regulate the genes encoding the above enzymes involved.

In one embodiment, the regulatory element is selected from an M1-93 artificial regulatory element, an MRS1 artificial regulatory element, a RBS artificial regulatory element or an M1-46 artificial regulatory element.

In one embodiment, the M1-93 artificial regulatory element regulates the *ilvD, leuDH, ilvBN, zwf, pgl,* and *ilvGM* genes.

In one embodiment, the MRS1 artificial regulatory element regulates an *edd* gene.

In one embodiment, the RBS artificial regulatory element regulates a gene *eda.*

In one embodiment, the M1-46 artificial regulatory element regulates an *ilvC* gene*.*

The regulatory element may be inserted at an upstream of the gene by a known genetic engineering method. The method includes, but is not limited to, inserting the sequence of the regulatory element into the upstream of the gene encoding sequence of the target enzyme by means of gene recombination, for example, by means of homologous recombination, so as to enhance the intensity of the target gene expression.

In one embodiment, herein the enzyme encoding gene and the regulatory element are integrated into the genome of the microorganism.

In one embodiment, a plasmid containing the enzyme encoding gene and the regulatory element sequence is transferred into the microorganism.

In one embodiment, the transfer, mutation or knockout of the target enzyme gene is completed by a method of integrating into the genome of the microorganism.

In one embodiment, the transfer, mutation or knockout of the enzyme gene is completed by a method of homologous recombination.

In one embodiment, the transfer, mutation or knockout of the enzyme gene is completed by a method of two-step homologous recombination.

A homologous recombination system known in the field may be used, such as an *Escherichia coli* RecA recombination system, and the Red recombination system is used for the homologous recombination to achieve the transfer, mutation or knockout of the target gene.

The two-step homologous recombination method to introduce, mutate or knock out the target gene includes the following steps *(Escherichia coli are* taken as an example):
(1) Preparation of a DNA fragment I: a pXZ-CS plasmid (Tan, et al., Appl Environ Microbiol, 2013, 79:4838-4844) DNA is used as a template, an amplification primer 1 is used to amplify the DNA fragment I*,* and used for the first step of homologous recombination;
(2) the first step of the homologous recombination: a pKD46 plasmid (Datsenko and Wanner 2000, Proc Natl Acad Sci USA 97:6640-6645) is transformed into *Escherichia coli,* and then the DNA fragment I is transformed into the *Escherichia coli* containing pKD46, a detection primer 1 is used to verify the transformed bacteria and select the correct colony;
(3) preparation of a DNA fragment II: the original *Escherichia coli* are used as a template, and an amplification primer 2 is used to amplify the DNA fragment II. The DNA fragment II is used for a second step of homologous recombination; and
(4) the second step of the homologous recombination: the DNA fragment II is transformed into the strain obtained in the second step; and a detection primer 2 is used to verify the transformed bacteria and select a correct colony.

The second aspect of the disclosure provides a recombinant microorganism for producing L-valine obtained by using the above construction method, specifically recombinant *Escherichia coli,* it contains an acetohydroxy acid isoreductase and/or an amino acid dehydrogenase gene,

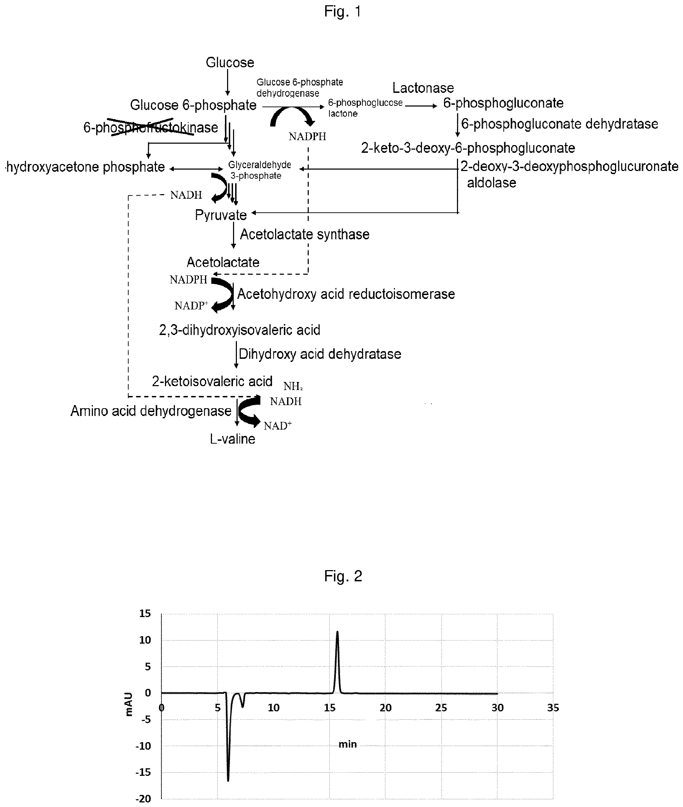

In one embodiment, the *Escherichia coli ATCC* 8739 is used as an original strain, and coupling of intracellular cofactor NADH supply and cell growth is achieved through the gene homologous recombination, thereby coupling (Fig. 1) of the cell growth and L-valine production under anaerobic conditions is achieved.

In one embodiment, the recombinant *Escherichia coli* obtained by the above construction method undergo metabolic evolution, for example, through 50 generations, 70 generations, 80 generations, 90 generations, 100 generations, and 120 generations, the recombinant *Escherichia coli* for highly producing L-valine is obtained. In one embodiment, a recombinant *Escherichia coli* strain producing L-valine is obtained after 70 generations of metabolic evolution, the deposition number thereof is: CGMCC 19457, and the classification name is: *Escherichia coli,* a biological material is submitted for deposited on March 6, 2020, Depository Authority: China General Microbiological Culture Collection Center (CGMCC), and the deposited address is No.3, Courtyard 1, Beichen West Road, Chaoyang District, Beijing.

The third aspect of the disclosure is an application of the recombinant microorganism obtained by the above method in producing L-valine.

The fourth aspect of the disclosure is a method for fermentatively producing L-valine using the recombinant microorganism obtained by the above construction method, including: (1) fermenting the recombinant microorganism obtained by the above construction method; and (2) separating and harvesting L-valine.

In one embodiment, the fermentation is anaerobic fermentation.

In one embodiment, the anaerobic fermentation includes the following steps:
(1) seed culture: a clone on a plate is picked and inoculated into a seed culture medium at 37°C, and shake culture is performed, to obtain seed culture solution; and
(2) fermentation culture: the seed culture solution is inoculated into fermentation culture medium, and culturing at 37°C and 150 rpm for 4 days, to obtain fermentation solution. The pH during fermentation process is controlled at 7.0. No air was sparged during the fermentation.

Herein the seed culture medium is formed by the following components (a solvent is water):
Glucose 20 g/L, corn syrup dry powder 10 g/L, KH₂PO4 8.8 g/L, (NH₄)₂SO₄ 2.5 g/L, and MgSO₄ •7H₂O 2 g/L.

The fermentation culture medium and the seed culture medium have the same components, and a difference is only that the glucose concentration is 50 g/L.

The beneficial effects of the disclosure:
(1) Compared with previous production methods and strains, the disclosure achieves the one-step anaerobic fermentation production of L-valine, reduces the production cost and improves the transformation rate.
(2) The disclosure preferably constructs a genetically stable L-valine production strain by modified directly on the genome of the recombinant microorganism rather than in the form of plasmid, and additional addition of substances such as antibiotics and inducers does not required, and the production process is stable and easy to operate.
(3) Through metabolic evolution, the titer and yield of L-valine and cell tolerance are improved significantly in the recombinant microorganisms.

### Preservation of Biological Material

Recombinant *Escherichia coli* Sval049 constructed in the disclosure are classified and named: *Escherichia coli.* A biological material is submitted for preservation on March 6, 2020, Depository Authority: China General Microbiological Culture Collection Center (CGMCC), the deposit address is No.3, Courtyard 1, Beichen West Road, Chaoyang District, Beijing, Telephone: 010-64807355, and the deposit number is CGMCC No. 19457.

### Brief Description of the Drawings

Drawings of the description for constituting a part of the present disclosure are used to provide further understanding of the disclosure. Exemplary embodiments of the disclosure and descriptions thereof are used to explain the disclosure, and do not constitute improper limitation to the disclosure. In the drawings:
Fig. 1: L-valine synthesis pathway.
Fig. 2: Determination of a standard substance of L-valine by high performance liquid chromatography.
Fig. 3: Determination of fermentation solution components of strain Sval048 by high performance liquid chromatography.
Fig. 4: construction of strain Sval049 by metabolic evolution.
Fig. 5: Determination of a standard substance of L-valine by high performance liquid chromatography.
Fig. 6: Determination of fermentation solution components of strain Sval049 by high performance liquid chromatography.

### Detailed Description of the Embodiments

The disclosure is further described by the following embodiments, but any embodiments or combinations thereof should not be interpreted as limiting a scope or an embodiment of the disclosure. The scope of the disclosure is defined by appended claims. In combination with the description and common knowledge in the field, those of ordinary skill in the art may clearly understand the scope defined by the claims. Without departing from the spirit and scope of the disclosure, those skilled in the art may make any modifications or changes to technical schemes of the disclosure, and such modifications and changes are also included in the scope of the disclosure.

Experimental methods used in the following embodiments are conventional methods unless otherwise specified. Materials, reagents and the like used in the following embodiments may be obtained from commercial sources unless otherwise specified.

Strains and plasmids constructed in this research are shown in Table 1, and primers used are shown in Table 2.

**Table 1: Strains and plasmids used in the disclosure**

| **Strain** | **Related characteristics** | **Sources** |
|---|---|---|
| ATCC 8739 | Wild type | Laboratory deposit |
| M1-93 | ATCC 8739, FRT-Km-FRT::M1-93::lacZ | Lu, et al., Appl Microbiol Biotechnol, 2012,93:2455-2462 |
| M1-46 | ATCC 8739, FRT-Km-FRT::M1-46::lacZ | Lu, et al., Appl Microbiol Biotechnol, 2012,93:2455-2462 |
| M1-37 | ATCC 8739, FRT-Km-FRT::M1-37::lacZ | Lu, et al., Appl Microbiol Biotechnol, 2012,93:2455-2462 |
| Sval001 | ATCC 8739, mgsA::cat-sacB | Constructed by the disclosure |
| Sval002 | Sval001, ΔmgsA | Constructed by the disclosure |
| Sval003 | Sval002, ldhA::cat-sacB | Constructed by the disclosure |
| Sval004 | Sval003, ΔldhA | Constructed by the disclosure |
| Sval005 | Sval004, ackA-pta::cat-sacB | Constructed by the disclosure |
| Sval006 | Sval005, Δ ackA-pta | Constructed by the disclosure |
| Sval007 | Sval006, tdcDE::cat-sacB | Constructed by the disclosure |
| Sval008 | Sval007, ΔtdcDE | Constructed by the disclosure |
| Sval009 | Sval008, adhE::cat-sacB | Constructed by the disclosure |
| Sval010 | Sval009, ΔadhE | Constructed by the disclosure |
| Sval011 | Sval010, mgsA::cat-sacB | Constructed by the disclosure |
| Sval012 | Sval011, mgsA::ilvC | Constructed by the disclosure |
| Sval013 | Sval012, mgsA::cat-sacB::ilvC | Constructed by the disclosure |
| Sval014 | Sval013, mgsA::M1-46-ilvC | Constructed by the disclosure |
| Sval015 | Sval014, pflB::cat-sacB | Constructed by the disclosure |
| Sval016 | Sval015, pflB::ilvD | Constructed by the disclosure |
| Sval017 | Sval016, pflB::cat-sacB::ilvD | Constructed by the disclosure |
| Sval018 | Sval017, pflB::RBS4-ilvD | Constructed by the disclosure |
| Sval019 | Sval018, cat-sacB::ilvB | Constructed by the disclosure |
| Sval020 | Sval019, M1-93:: ilvB | Constructed by the disclosure |
| Sval021 | Sval020, cat-sacB::ilvG | Constructed by the disclosure |
| Sval022 | Sval021, M1-93:: ilvG | Constructed by the disclosure |
| Sval023 | Sval022, ilvH::cat-sacB | Constructed by the disclosure |
| Sval024 | Sval023, ilvH:: ilvH* | Constructed by the disclosure |
| Sval025 | Sval024, frd::cat-sacB | Constructed by the disclosure |
| Sval026 | Sval025, frd::M1-93-leuDH | Constructed by the disclosure |
| Sval041 | Sval026, cat-sacB::zwf | Constructed by the disclosure |
| Sval042 | Sval041, RBS1-zwf | Constructed by the disclosure |
| Sval043 | Sval042, cat-sacB::pgl | Constructed by the disclosure |
| Sval044 | Sval043, RBS2-pgl | Constructed by the disclosure |
| Sval045 | Sval044, edd-eda::cat-sacB | Constructed by the disclosure |
| Sval046 | Sval045,edd-eda::MRS1 -edd-eda | Constructed by the disclosure |
| Sval047 | Sval046, pfkA::cat-sacB | Constructed by the disclosure |
| Sval048 | Sval047, ΔpfkA | Constructed by the disclosure |
| Sval049 | metabolic evolution of of Sval048 for 100 generations, CGMCC 19457 | Constructed by the disclosure |

| **Plasmid** | **Related characteristics** | **Sources** |
|---|---|---|
| pUC57-M1-93-leuDH | Artificial regulatory element M1-93 and chemically synthesized gene leuDH are linked to a pUC57 vector together | Nanjing Genscript Biotechnology Co., Ltd. |
| pUC57-MRS1-edd-eda | Artificial regulatory element MRS1 and chemically synthesized genes edd and eda are linked to the pUC57 vector together | Nanjing Genscript Biotechnology Co., Ltd. |

**Table 2: Primers used in the disclosure**

| **Primer name** | **Sequence** | **Sequenc e number** |
|---|---|---|
| mgsA-cs-up | gtaggaaagttaactacggatgtacattatggaactgacgactcgcacttTGTGA CGGAAGATCACTTCGCAG | 1 |
| mgsA-cs-dow n | gcgtttgccacctgtgcaatattacttcagacggtccgcgagataacgctTTATTT GTTAACTGTTAATTGTCCT | 2 |
| XZ-mgsA-up | cagctcatcaaccag gtcaa | 3 |
| XZ-mgsA-do wn | aaaagccgtcacgttattg g | 4 |
| mgsA-del-do wn | Gcgtttgccacctgtgcaatattacttcagacggtccgcgagataacgctaagtgcg agtcgtcagttcc | 5 |
| mgsA-ilvC-up | gtaggaaagttaactacggatgtacattatggaactgacgactcgcacttATGGC TAACTACTTCAATACac | 6 |
| mgsA-ilvC-do wn | gcgtttgccacctgtgcaatattacttcagacggtccgcgagataacgctTTAACC CGCAACAGCAATACGtttc | 7 |
| mgsA-Pcs-up | gtaggaaagttaactacggatgtacattatggaactgacgactcgcacttTGTGA CGGAAGATCACTTCGCAG | 8 |
| mgsA-Pcs-do wn | agctgtgccagctgctggcgcagattcagtGTATTGAAGTAGTTAGCCA TTTATTTGTTAACTGTTAATTGTCCT | 9 |
| mgsA-P46-u p | gtaggaaagttaactacggatgtacattatggaactgacgactcgcacttTTATCT CTGGCGGTGTTGAC | 10 |
| ilvC-P46-dow n | agctgtgccagctgctggcgcagattcagtGTATTGAAGTAGTTAGCCA TAGCTGTTTCCTGGTTTAAACCG | 11 |
| ilvC-YZ347-d own | cgcactacatcagagtgctg | 12 |
| ldhA-cs-up | ttcaacatcactggagaaagtcttatgaaactcgccgtttatagcacaaaTGTGA CGGAAGATCACTTCGCAG | 13 |
| ldhA-cs-down | agcggcaagattaaaccagttcgttcgggcaggtttcgcctttttccagaTTATTT GTTAACTGTTAATTGTCCT | 14 |
| XZ-ldhA-up | GATAACGGAGATCGGGAATG | 15 |
| XZ-ldhA-down | CTTTGGCTGTCAGTTCACCA | 16 |
| ldhA-del-dow n | agcggcaagattaaaccagttcgttcgggcaggtttcgcctttttccagatttgtgctat aaacggcgagt | 17 |
| ackA-cs-up | aggtacttccatgtcgagtaagttagtactggttctgaactgcggtagttTGTGAC GGAAGATCACTTCGCAG | 18 |
| pta-cs-down | ggtcggcagaacgctgtaccgctttgtaggtggtgttaccggtgttcagaTTATTT GTTAACTGTTAATTGTCCT | 19 |
| XZ-ackA-up | cgggacaacgttcaaaacat | 20 |
| XZ-pta-down | attgcccatcttcttgttgg | 21 |
| ackA-del-dow n | ggtcggcagaacgctgtaccgctttgtaggtggtgttaccggtgttcagaaactaccg cagttcagaacca | 22 |
| tdcDE-cs-up | ccgtgattggtctgctgaccatcctgaacatcgtatacaaactgttttaaTGTGAC GGAAGATCACTTCGCAG | 23 |
| tdcDE-cs-do wn | cgcctggggcacgttgcgtttcgataatctttttcatacatcctccggcgTTATTTG TTAACTGTTAATTGTCCT | 24 |
| XZ-tdcDE-up | TGATGAGCTACCTGGTATGGC | 25 |
| XZ-tdcDE-do wn | CGCCGACAGAGTAATAGGTTTTAC | 26 |
| tdcDE-del-do wn | cgcctggggcacgttgcgtttcgataatctttttcatacatcctccggcgttaaaacagt ttgtatacgatgttcag | 27 |
| adhE-cs-up | ATAACTCTAATGTTTAAACTCTTTTAGTAAATCACAGTGAG TGTGAGCGCTGTGACGGAAGATCACTTCGCA | 28 |
| adhE-cs-dow n | CCGTTTATGTTGCCAGACAGCGCTACTGATTAAGCGGATT TTTTCGCTTTTTATTTGTTAACTGTTAATTGTCCT | 29 |
| adhE-del-do wn | CCGTTTATGTTGCCAGACAGCGCTACTGATTAAGCGGATT TTTTCGCTTTGCGCTCACACTCACTGTGATTTAC | 30 |
| XZ-adhE-up | CATGCTAATGTAGCCACCAAA | 31 |
| XZ-adhE-do wn | TTGCACCACCATCCAGATAA | 32 |
| pflB-CS-up | aaacgaccaccattaatggttgtcgaagtacgcagtaaataaaaaatccaTGTG ACGGAAGATCACTTCGCAG | 33 |
| pflB-CS-dow n | CGGTCCGAACGGCGCGCCAGCACGACGACCGTCTGGG GTGTTACCCGTTTTTATTTGTTAACTGTTAATTGTCCT | 34 |
| pflB-ilvD-up | aaacgaccaccattaatggttgtcgaagtacgcagtaaataaaaaatccaatgcct aagtaccgttccgc | 35 |
| pflB-ilvD-dow n | CGGTCCGAACGGCGCGCCAGCACGACGACCGTCTGGG GTGTTACCCGTTTttaaccccccagtttcgatttatc | 36 |
| XZ-pflB-up60 0 | CTGCGGAGCCGATCTCTTTAC | 37 |
| XZ-pflB-down | CGAGTAATAACGTCCTGCTGCT | 38 |
| pflB-Pcs-up | aaacgaccaccattaatggttgtcgaagtacgcagtaaataaaaaatccaTGTG ACGGAAGATCACTTCGCA | 39 |
| pflB-Pcs-dow n | CCCGCCATATTACGACCATGAGTGGTGGTGGCGGAACGG TACTTAGGCATTTATTTGTTAACTGTTAATTGTCCT | 40 |
| pflB-Pro-up | AAACGACCACCATTAATGGTTGTCGAAGTACGCAGTAAAT AAAAAATCCATTATCTCTGGCGGTGTTGAC | 41 |
| ilvD-Pro-dow n | cccgccatattacgaccatgagtggtggtggcggaacggtacttaggcatTGCT GACCTCCTGGTTTAAACGTACATG | 42 |
| ilvD-YZ496-d own | caaccagatcgagcttgatg | 43 |
| XZ-frd-up | TGCAGAAAACCATCGACAAG | 44 |
| XZ-frd-down | CACCAATCAGCGTGACAACT | 45 |
| frd-cs-up | GAAGGCGAATGGCTGAGATGAAAAACCTGAAAATTGAGG TGGTGCGCTATTGTGACGGAAGATCACTTCGCA | 46 |
| frd-cs-down | TCTCAGGCTCCTTACCAGTACAGGGCAACAAACAGGATT ACGATGGTGGCTTATTTGTTAACTGTTAATTGTCCT | 47 |
| frd-M93-up | GAAGGCGAATGGCTGAGATGAAAAACCTGAAAATTGAGG TGGTGCGCTATTTATCTCTGGCGGTGTTGAC | 48 |
| frd-leuDH-do wn | TCTCAGGCTCCTTACCAGTACAGGGCAACAAACAGGATT ACGATGGTGGCTTAACGGCCGTTCAAAATATTTTTTTC | 49 |
| ilvB pro-catup | ctgacgaaacctcgctccggcggggtttttgttatctgcaattcagtacTGTGAC GGAAGATCACTTCGCA | 50 |
| ilvB pro-catdown | tctgcgccggtaaagcgcttacgcgtcgatgttgtgcccgaacttgccatTTATTT GTTAACTGTTAATTGTCCT | 51 |
| ilvB pro-up | ctgacgaaacctcgctccggcggggtttttgttatctgcaattcagtacTTATCTC TGGCGGTGTTGAC | 52 |
| ilvB pro-down | tctgcgccggtaaagcgcttacgcgtcgatgttgtgcccgaacttgccatAGCTG TTTCCTGGTTTAAAC | 53 |
| ilvB pro-YZup | gttctgcgcgg aacacgtatac | 54 |
| ilvB pro- YZdown | ccgctacag gccatacag ac | 55 |
| ilvG pro-catup | tgaactaagaggaagggaacaacattcagaccgaaattgaatttttttcaTGTGA CGGAAGATCACTTCGCA | 56 |
| ilvG pro-catdown | ttcacaccctgtgcccgcaacgcatgtaccacccactgtgcgccattcatTTATTT GTTAACTGTTAATTGTCCT | 57 |
| ilvG pro-up | tgaactaagaggaagggaacaacattcagaccgaaattgaatttttttcaTTATC TCTGGCGGTGTTGAC | 58 |
| ilvG pro-down | ttcacaccctgtgcccgcaacgcatgtaccacccactgtgcgccattcatAGCTG TTTCCTGGTTTAAACG | 59 |
| ilvG pro-YZup | gcataagatatcgctgctgtag | 60 |
| ilvG | gccagttttgccagtagcac | 61 |
| p-YZdown | | |
| ilvH*-cat-up | agaacctgattatgCGCCGGATATTATCAGTCTTACTCGAAAATG AATCATGTGACGGAAGATCACTTCGCA | 62 |
| ilvH*-cat-dow n | TTCATCGCCCACGGTCTGGATGGTCATACGCGATAATGTC GGATCGTCGGTTATTTGTTAACTGTTAATTGTCCT | 63 |
| ilvH*-mut-up | agaacctgattatgCGCCGGATATTATCAGTCTTACTCGAAAATG AATCAGaCGCGTTATtCCGCGTGATTGGC | 64 |
| ilvH*-mut-do wn | CACACCAGAGCGAGCAACCTC | 65 |
| ilvH*-mutYZ-up | atg agctgg aaagcaaacttagc | 66 |
| Zwf-Pcat-up | agttttgccgcactttgcgcgcttttcccgtaatcgcacgggtggataagTGTGAC GGAAGATCACTTCGCA | 67 |
| Zwf-PsacB-d own | gcgccgaaaatgaccaggtcacaggcctgggctgtttgcgttaccgccatTTATT TGTTAACTGTTAATTGTCCT | 68 |
| zwf-RBS1-up | agttttgccgcactttgcgcgcttttcccgtaatcgcacgggtggataagTTATCTC TGGCGGTGTTGAC | 69 |
| zwf-RBS1-do wn | gcgccgaaaatgaccaggtcacaggcctgggctgtttgcgttaccgccatATTG TTTCTCCTGGTTTAAACGTACGTG | 70 |
| zwf-YZ442-u P | cgaatggatcgcgttatcgg | 71 |
| zwf-YZ383-d own | caaattgcgccaaaagtgctg | 72 |
| pgl-Pcat-up | ttcagcattcaccgccaaaagcgactaattttagctgttacagtcagttgTGTGAC GGAAGATCACTTCGCA | 73 |
| pgl-PsacB-do wn | acgtgaatttgctggctctcagggctggcgatataaactgtttgcttcatTTATTTG TTAACTGTTAATTGTCCT | 74 |
| pgl-RBS2-up | ttcagcattcaccgccaaaagcgactaattttagctgttacagtcagttgTTATCTC TGGCGGTGTTGAC | 75 |
| pgl-RBS2-do wn | acgtgaatttgctggctctcagggctggcgatataaactgtttgcttcatACGTTTC CTCCTGGTTTAAACGTACATGCTAACAATAC | 76 |
| pgl-YZ308-up | gatgaatagcgacgtgatgg | 77 |
| pgl-YZ341-do wn | ccatcttccag acgcgttac | 78 |
| Edd-cat-up | tggtcgttcctggaatgagtttgagtaatatctgcgcttatcctttatggTGTGACG GAAGATCACTTCGCA | 79 |
| Eda-sacB-do wn | gcaaaaaaacgctacaaaaatgcccgatcctcgatcgggcattttgacttTTATT TGTTAACTGTTAATTGTCCT | 80 |
| Edd-int-up | tggtcgttcctggaatgagtttgagtaatatctgcgcttatcctttatggTTATCTCT GGCGGTGTTGAC | 81 |
| Eda-int-down | gcaaaaaaacgctacaaaaatgcccgatcctcgatcgggcattttgacttTTAG GCAACAGCAGCGCGCTTG | 82 |
| Edd-YZ-up | gcatctggcggatgcctatg | 83 |
| Edd-YZ-dow n | caactgaccagtcagaatgtcac | 84 |
| pfkAdel-cat-u p | GGTATCGACGCGCTGGTGGTTATCGGCGGTGACGGTTC CTACATGGGTGCTGTGACGGAAGATCACTTCGCA | 85 |
| pfkAdel-sacB -down | GTGGCCCAGCACAGTTGCGCGGGTTTCACGACCGGTTT CTTTCTCGATGATTATTTGTTAACTGTTAATTGTCCT | 86 |
| pfkAdel-up | ATGATTAAGAAAATCGGTGTG | 87 |
| pfkAdel-down | GTGGCCCAGCACAGTTGCGCGGGTTTCACGACCGGTTT CTTTCTCGATGAGCACCCATGTAGGAACCGTC | 88 |
| pfkAdel-YZ-d own | GTCGATGATGTCGTGGTGAAC | 89 |

### Example 1: Knockout of methylglyoxal synthase encoding gene mgsA in ATCC 8739 strain

Started from *Escherichia coli ATCC* 8739, a two-step homologous recombination method is used to knock out the methylglyoxal synthase encoding gene *mgsA,* and specific steps are as follows.

In a first step, a pXZ-CS plasmid DNA is used as a template, 2719 bp of a DNA fragment I is amplified by using primers mgsA-cs-up/mgsA-cs-down, and used for the first step of homologous recombination.

An amplification system is: Phusion 5X buffer (NewEngland Biolabs) 10 µl, dNTP (10 mM for each dNTP) 1 µl, DNA template 20 ng, primers (10 µM) 2 µl each, Phusion High-Fidelity DNA polymerase (NewEngland Biolabs) (2.5 U/µl) 0.5 µl, distilled water 33.5 µl, and a total volume is 50 µl. Amplification conditions are 98°C pre-denaturation for 2 minutes (1 cycle); 98°C denaturation for 10 seconds, 56°C annealing for 10 seconds, 72°C extension for 2 minutes (30 cycles); and 72°C extension for 10 minutes (1 cycle).

The above DNA fragment I is used for the first homologous recombination: firstly, a pKD46 plasmid (purchased from the Coil Genetic Stock Center (CGSC) of Yale University, CGSC#7739) is transformed into *Escherichia coli* ATCC 8739 by an electrotransformation method, and then the DNA fragment I is electrotransformed to the *Escherichia coli ATCC* 8739 with the pKD46.

Electrotransformation conditions are as follows: firstly, electrotransformation competent cells of the *Escherichia coli ATCC* 8739 with the pKD46 plasmid are prepared; 50 µl of the competent cells are placed on ice, and 50 ng of the DNA fragment I is added. The mixture was placed on ice for 2 minutes, and transferred into a 0.2 cm MicroPulser Electroporation Cuvette (Bio-Rad). The electroporation was carried with the MicroPulser (Bio-Rad) electroporation apparatus and the electric voltage was 2.5 kV. After electric shock, 1 ml of LB medium was quickly added into the electroporation cuvette, and transferred into a test tube after pipetting five times. The culture was incubated at 30°C with shaking at 75 rpm for 2 hours. 200 µl of culture was spread onto a LB plate containing ampicillin (a final concentration is 100 µg/ml) and chloramphenicol (a final concentration is 34 µg/ml). After being cultured overnight at 30°C, colonies were verified with primer set XZ-mgsA-up/XZ-mgsA-down, and a correct colony amplification product is a 3646 bp fragment. A correct single colony was selected, and named as Sval001.

In a second step, a genomic DNA of wild-type *Escherichia coli ATCC* 8739 is used as template, and 566 bp of a DNA fragment II is amplified with primer set XZ-mgsA-up/mgsA-del-down. DNA fragment II is used for the second homologous recombination. Amplification conditions and system are the same as those described in the first step. The DNA fragment II is electrotransformed into strain Sval001.

Electrotransformation conditions are as follows: firstly, electrotransformation competent cells of the Sval001 with the pKD46 plasmid (Dower et al., 1988, Nucleic Acids Res 16:6127-6145) were prepared; 50 µl of the competent cells were placed on ice, and 50 ng of a DNA fragment II is added. The mixture was placed on ice for 2 minutes, and transferred into a 0.2 cm MicroPulser Electroporation Cuvette (Bio-Rad). The electroporation was carried with the MicroPulser (Bio-Rad) electroporation apparatus and the electric voltage was 2.5 kV. After electric shock, 1 ml of LB medium was quickly added into the electroporation cuvette, and transferred into a test tube after pipetting five times. The culture was incubated at 30°C with shaking at 75 rpm for 4 hours. The culture was then transferred into LB medium containing 10% sucrose but without a sodium chloride

(50 ml of a medium is loaded in 250 ml of a flask), and after being cultured for 24 hours, it is streak-cultured on an LB solid medium containing 6% sucrose without a sodium chloride. The correct clone was verified by colony PCR amplification with primer set XZ-mgsA-up/XZ-mgsA-down, and a correct colony amplification product was 1027 bp. A correct single colony is then selected, and named as Sval002 (Table 1).

### Example 2: Knockout of lactate dehydrogenase encoding gene ldhA

Started from Sval002, and a lactate dehydrogenase encoding gene *ldhA* is knocked out by a two-step homologous recombination method. Specific steps are as follows.

In a first step, a pXZ-CS plasmid DNA is used as a template, 2719 bp of a DNA fragment I is amplified by using primers ldhA-cs-up/ldhA-cs-down, and used for the first step of the homologous recombination. Amplification system and amplification conditions are the same as those described in Example 1. The DNA fragment I is electrotransformed to the Sval002.

The DNA fragment I is used for the first homologous recombination: firstly, a pKD46 plasmid is transformed into *Escherichia coli* Sval002 by an electrotransformation method, and then the DNA fragment I is electrotransformed into the *Escherichia coli* Sval002 with the pKD46.

Electrotransformation conditions and steps are the same as the first step method for the *mgsA* gene knockout described in Example 1. 200 µl of culture solution is spreaded onto a LB plate containing ampicillin (a final concentration is 100 µg/ml) and chloramphenicol (a final concentration is 34 µg/ml). After being cultured overnight at 30°C, colonies were PCR verified with primer set XZ-ldhA-up/XZ-ldhA-down, and a correct PCR product should be 3448 bp. A correct single colony is picked, and named as Sval003.

In a second step, a DNA of wild-type *Escherichia coli ATCC* 8739 is used as a template, and 476 bp of a DNA fragment II is amplified with primers XZ-ldhA-up/ldhA-del-down. The DNA fragment II is used for the second homologous recombination. The DNA fragment II is electrotransformed into strain Sval003.

Electrotransformation conditions and steps are the same as the second step method for the *mgsA* gene knockout described in Example 1. Colony PCR is used to verify clones, the used primers are XZ-ldhA-up/XZ-ldhA-down, and a correct colony amplification product is 829 bp. A correct single colony is picked, and named as Sval004 (Table 1).

### Example 3: Knockout of phosphoacetyl transferase encoding gene pta and acetate kinase encoding gene ackA

Started from Sval004, a two-step homologous recombination method is used to knock out a phosphoacetyl transferase encoding gene *pta* and an acetate kinase encoding gene *ackA.* Specific steps are as follows.

In a first step, a pXZ-CS plasmid DNA is used as a template, 2719 bp of a DNA fragment I is amplified by using primers ackA-cs-up/pta-cs-down, and used for the first step of the homologous recombination. Amplification system and amplification conditions are the same as those described in Example 1. The DNA fragment I is electrotransformed to the Sval004.

The DNA fragment I is used for the first homologous recombination: firstly, a pKD46 plasmid is transformed into *Escherichia coli* Sval004 by an electrotransformation method, and then the DNA fragment I is electrotransformed into the *Escherichia coli* Sval004 with the pKD46.

Electrotransformation conditions and steps are the same as the first step method for the *mgsA* gene knockout described in Example 1. 200 µl of bacterial solution is spreadedonto an LB plate containing ampicillin (a final concentration is 100 µg/ml) and chloramphenicol (a final concentration is 34 µg/ml). After being cultured overnight at 30°C, colonies were PCR verified using primers XZ-ackA-up/XZ-pta-down, and a correct PCR product should be 3351 bp. A correct single colony is picked, and named as Sval005.

In a second step, a DNA of wild-type *Escherichia coli ATCC* 8739 is used as a template, and 371 bp of a DNA fragment II is amplified with primers XZ-ackA-up/ackA-del-down. The DNA fragment II is used for the second homologous recombination. The DNA fragment II is electrotransformed into strain Sval005.

Electrotransformation conditions and steps are the same as the second step method for the *mgsA* gene knockout described in Example 1. Colony PCR is used to verify clones using primers XZ-ackA-up/XZ-pta-down, and a correct colony amplification product is 732 bp. A correct single colony is picked, and named as Sval006 (Table 1).

### Example 4: Knockout of propionate kinase encoding gene tdcD and formate acetyltransferase encoding gene tdcE

Started from Sval006, a two-step homologous recombination method is used to knock out the propionate kinase encoding gene *tdcD* and the formate acetyltransferase encoding gene *tdcE.* Specific steps are as follows.

In a first step, a pXZ-CS plasmid DNA is used as a template, 2719 bp of a DNA fragment I is amplified by using primers tdcDE-cs-up/tdcDE-cs-down, and used for the first step of the homologous recombination. Amplification system and amplification conditions are the same as those described in Example 1. The DNA fragment I is electrotransformed to the Sval006.

The DNA fragment I is used for the first homologous recombination: firstly, a pKD46 plasmid is transformed into *Escherichia coli* Sval006 by an electrotransformation method, and then the DNA fragment I is electrotransformed into the *Escherichia coli* Sval006 with the pKD46.

Electrotransformation conditions and steps are the same as the first step method for the *mgsA* gene knockout described in Example 1. 200 µl of culture solution is spreaded onto a LB plate containing ampicillin (a final concentration is 100 µg/ml) and chloramphenicol (a final concentration is 34 µg/ml). After being cultured overnight at 30°C, colonies were PCR verified using primers XZ-tdcDE-up/ XZ-tdcDE-down, and a correct PCR product should be 4380 bp. A correct single colony is picked, and named as Sval007.

In a second step, a DNA of wild-type *Escherichia coli ATCC* 8739 is used as a template, and 895 bp of a DNA fragment II is amplified with primers XZ-tdcDE-up/tdcDE-del-down. The DNA fragment II is used for the second homologous recombination. The DNA fragment II is electrotransformed into strain Sval007.

Electrotransformation conditions and steps are the same as the second step method for the *mgsA* gene knockout described in Example 1. Colonies were PCR verified using primers XZ-tdcDE-up/ XZ-tdcDE-down, and a correct colony amplification product is 1761 bp . A correct single colony is picked, and named as Sval008 (Table 1).

### Example 5: Knockout of alcohol dehydrogenase gene adhE

Started from Sval008, a two-step homologous recombination method is used to knock out the alcohol dehydrogenase gene *adhE.* Specific steps are as follows.

In a first step, a pXZ-CS plasmid DNA is used as a template, 2719 bp of a DNA fragment I is amplified by using primers adhE-cs-up /adhE-cs-down, and used for the first step of the homologous recombination. Amplification system and amplification conditions are the same as those described in Example 1.

The DNA fragment I is used for the first homologous recombination: firstly, a pKD46 plasmid is transformed into *Escherichia coli* Sval008 by an electrotransformation method, and then the DNA fragment I is electrotransformed into the *Escherichia coli* Sval008 with the pKD46.

Electrotransformation conditions and steps are the same as the first step method for the *mgsA* gene knockout described in Example 1. 200 µl of bacterial solution is spreaded onto a LB plate containing ampicillin (a final concentration is 100 µg/ml) and chloramphenicol (a final concentration is 34 µg/ml). After being cultured overnight at 30°C, colonies were PCR verified using primers XZ-adhE-up/XZ-adhE-down, and a correct PCR product should be 3167 bp. A correct single colony is picked, and named as Sval009.

In a second step, a DNA of wild-type *Escherichia coli ATCC* 8739 is used as a template, and 271 bp of a DNA fragment II is amplified with primers XZ-adhE-up/adhE-del-down. The DNA fragment II is used for the second homologous recombination. The DNA fragment II is electrotransformed into strain Sval009.

Electrotransformation conditions and steps are the same as the second step method for the *mgsA* gene knockout described in Example 1. Colonies were PCR verified using primers XZ-adhE-up/XZ-adhE-down, and a correct colony amplification product is 548 bp . A correct single colony is picked, and named as Sval010 (Table 1).

### Example 6: Integration of acetohydroxy acid reductoisomerase encoding gene ilvC in methylglyoxal synthase encoding gene mgsA site

Started from Sval010, an acetohydroxy acid reductoisomerase encoding gene *ilvC* from *Escherichia coli* is integrated into the methylglyoxal synthase encoding gene *mgsA* site through a two-step homologous recombination method. Specific steps are as follows.

In a first step, a cat-sacB fragment is integrated into the *mgsA* site of strain Sval010. PCR, integration, and verification of the cat-sacB fragment are exactly the same as the first step of the *mgsA* gene knockout in Example 1, and an obtained clone is named as Sva!011.

In a second step, a DNA of wild-type *Escherichia coli ATCC* 8739 is used as a template, 1576 bp of a DNA fragment II is amplified by using primers mgsA-ilvC-up/mgsA-ilvC-down. The DNA fragment II is used for the second homologous recombination. The DNA fragment II is electrotransformed into strain Sval011.

Electrotransformation conditions and steps are the same as the second step method for the *mgsA* gene knockout described in Example 1. Colonies were PCR verified using primers XZ-mgsA-up/XZ-mgsA-down and sequenced, and a correct colony amplification product is 2503 bp . A correct single colony is picked, and named as Sval012 (Table 1).

### Example 7: Regulation of acetohydroxy acid reductoisomerase encoding gene ilvC

Started from Sval012, and an artificial regulatory element is used to regulate expression of the acetohydroxy acid reductoisomerase encoding gene *ilvC* integrated in a methylglyoxal synthase encoding gene *mgsA* site. Specific steps are as follows.

In a first step, a pXZ-CS plasmid DNA is used as a template, 2719 bp of a DNA fragment I is amplified by using primers mgsA-Pcs-up/mgsA-Pcs-down, and used for the first step of the homologous recombination. Amplification system and amplification conditions are the same as those described in Example 1. The DNA fragment I is electrotransformed into the Sval012.

The DNA fragment I is used for the first homologous recombination: firstly, a pKD46 plasmid (purchased from the Coil Genetic Stock Center (CGSC) of Yale University, CGSC#7739) is transformed into *Escherichia coli* Sval012 by an electrotransformation method, and then the DNA fragment I is electrotransformed into the *Escherichia coli* Sval012 with the pKD46.

Electrotransformation conditions and steps are the same as the first step method for the *mgsA* gene knockout described in Example 1. 200 µl of bacterial solution is spreaded onto a LB plate containing ampicillin (a final concentration is 100 µg/ml) and chloramphenicol (a final concentration is 34 µg/ml). After being cultured overnight at 30°C, colonies were PCR verified using primers XZ-mgsA-up/ilvC-YZ347-down, and a correct PCR product should be 3482 bp. A correct single colony is picked, and named as Sval013.

In a second step, a genomic DNA of M1-46 (Lu, et al., Appl Microbiol Biotechnol, 2012,93:2455-2462) is used as a template, and 188 bp of a DNA fragment II is amplified by using primers mgsA-P46-up/ilvC-P46-down. The DNA fragment II is used for the second homologous recombination. The DNA fragment II is electrotransformed into strain Sval013.

Electrotransformation conditions and steps are the same as the second step method for the *mgsA* gene knockout described in Example 1. Colonies were PCR verified using primers XZ-mgsA-up/ilvC-YZ347-down and sequenced, and a correct colony amplification product is 951 bp . A correct single colony is picked, and named as Sval014 (Table 1).

### Example 8: Integration of dihydroxy acid dehydratase encoding gene ilvD

Started from Sval014, a dihydroxy acid dehydratase encoding gene *ilvD* from *Escherichia coli* is integrated into the pyruvate formate lyase encoding gene *pflB* site and replaces the *pflB* gene through a two-step homologous recombination method, namely the *pflB* gene is knocked out while the *ilvD* is integrated. Specific steps are as follows.

In a first step, a pXZ-CS plasmid DNA is used as a template, 2719 bp of a DNA fragment I is amplified by using primers pflB-CS-up/pflB-CS-down, and used for the first step of the homologous recombination. Amplification system and amplification conditions are the same as those described in Example 1. The DNA fragment I is electrotransformed into the Sval014.

The DNA fragment I is used for the first homologous recombination: firstly, a pKD46 plasmid is transformed into *Escherichia coli* Sval014 by an electrotransformation method, and then the DNA fragment I is electrotransformed into the *Escherichia coli* Sval014 with the pKD46.

Electrotransformation conditions and steps are the same as the first step method for the *mgsA* gene knockout described in Example 1. 200 µl of bacterial solution is spreaded onto a LB plate containing ampicillin (a final concentration is 100 µg/ml) and chloramphenicol (a final concentration is 34 µg/ml). After being cultured overnight at 30°C, colonies were PCR verified using primers XZ-pflB-up600/XZ-pflB-down, and a correct PCR product should be 3675 bp. A correct single colony is picked, and named as Sval015.

In a second step, a genomic DNA of *Escherichia coli* MG1655 (from ATCC, No. 700926) is used as a template, and 1951 bp of a DNA fragment II is amplified by using primers pflB-ilvD-up/pflB-ilvD-down. The DNA fragment II is used for the second homologous recombination. The DNA fragment II is electrotransformed into strain Sval015.

Electrotransformation conditions and steps are the same as the second step method for the *mgsA* gene knockout described in Example 1. Colonies were PCR verified using primers XZ-pflB-up600/XZ-pflB-down and sequenced, and a correct colony amplification product is 2907 bp . A correct single colony is picked, and named as Sval016 (Table 1).

### Example 9: Regulation of dihydroxy acid dehydratase encoding gene ilvD

Started from Sval016, and an artificial regulatory element is used to regulate expression of the dihydroxy acid dehydratase encoding gene *ilvD* integrated in the pyruvate formate lyase encoding gene *pflB* site. Specific steps are as follows.

In a first step, a pXZ-CS plasmid DNA is used as a template, 2719 bp of a DNA fragment I is amplified by using primers pflB-Pcs-up/pflB-Pcs-down, and used for the first step of the homologous recombination. Amplification system and amplification conditions are the same as those described in Example 1. The DNA fragment I is electrotransformed into the Sval016.

The DNA fragment I is used for the first homologous recombination: firstly, a pKD46 plasmid is transformed into *Escherichia coli* Sval016 by an electrotransformation method, and then the DNA fragment I is electrotransformed into the *Escherichia coli* Sval016 with the pKD46.

Electrotransformation conditions and steps are the same as the first step method for the *mgsA* gene knockout described in Example 1. 200 µl of bacterial solution is spreaded onto a LB plate containing ampicillin (a final concentration is 100 µg/ml) and chloramphenicol (a final concentration is 34 µg/ml). After being cultured overnight at 30°C, colonies were PCR verified using primers XZ-pflB-up600/ilvD-YZ496-down, and a correct PCR product should be 3756 bp. A correct single colony is picked, and named as Sval017.

In a second step, a genomic DNA of M1-93 (Lu, et al., Appl Microbiol Biotechnol, 2012,93:2455-2462) is used as a template, and 189 bp of a DNA fragment II is amplified by using primers pflB-Pro-up/ilvD-Pro-down. The DNA fragment II is used for the second homologous recombination. The DNA fragment II is electrotransformed into strain Sval017.

Electrotransformation conditions and steps are the same as the second step method for the *mgsA* gene knockout described in Example 1. Colonies were PCR verified using primers XZ-pflB-up600/ilvD-YZ496-down and sequenced, and a correct colony amplification product is 1226 bp . A correct single colony is picked, and named as Sval018 (Table 1).

### Example 10: Regulation of acetolactate synthase gene ilvBN

An artificial regulatory element M1-93 is used to regulate expression of an acetolactate synthase gene *ilvBN* through a two-step homologous recombination method. Specific steps are as follows.

In a first step, a pXZ-CS plasmid DNA is used as a template, 2719 bp of a DNA fragment I is amplified by using primers ilvB pro-catup/ilvB pro-catdown, and used for the first step of the homologous recombination. Amplification system and amplification conditions are the same as those described in Example 1.

The DNA fragment I is used for the first homologous recombination: firstly, a pKD46 plasmid is transformed into *Escherichia coli* Sval018 by an electrotransformation method, and then the DNA fragment I is electrotransformed into the *Escherichia coli* Sval018 with the pKD46.

Electrotransformation conditions and steps are the same as the first step method for the *mgsA* gene knockout described in Example 1. 200 µl of bacterial solution is spreaded onto a LB plate containing ampicillin (a final concentration is 100 µg/ml) and chloramphenicol (a final concentration is 34 µg/ml). After being cultured overnight at 30°C, colonies were PCR verified using primers ilvB pro-YZup/ilvB pro-YZdown, and a correct PCR product should be 2996 bp. A correct single colony is picked, and named as Sval019.

In a second step, a genomic DNA of M1-93 is used as a template, and 188 bp of a DNA fragment II is amplified by using primers ilvB pro-up/ilvB pro-down. The DNA fragment II is used for the second homologous recombination. The DNA fragment II is electrotransformed into strain Sval019. Electrotransformation conditions and steps are the same as the second step method for the *mgsA* gene knockout described in Example 1. Colonies were PCR verified using primers ilvB pro-YZup/ilvB pro-YZdown, and a correct colony amplification product is 465 bp. A correct single colony is picked, and named as Sval020.

### Example 11: Regulation of acetolactate synthase gene ilvGM

An artificial regulatory element M1-93 is used to regulate expression of the acetolactate synthase gene *ilvGM* through a two-step homologous recombination method. Specific steps are as follows.

In a first step, a pXZ-CS plasmid DNA is used as a template, 2719 bp of a DNA fragment I is amplified by using primers ilvG pro-catup / ilvG pro-catdown, and used for the first step of the homologous recombination. Amplification system and amplification conditions are the same as those described in Example 1.

The DNA fragment I is used for the first homologous recombination: firstly, a pKD46 plasmid is transformed into *Escherichia coli* Sval020 by an electrotransformation method, and then the DNA fragment I is electrotransformed into the *Escherichia coli* Sval020 with the pKD46.

Electrotransformation conditions and steps are the same as the first step method for the *mgsA* gene knockout described in Example 1. 200 µl of bacterial solution is spreaded onto a LB plate containing ampicillin (a final concentration is 100 µg/ml) and chloramphenicol (a final concentration is 34 µg/ml). After being cultured overnight at 30°C, colonies were PCR verified using primers ilvG pro-YZup / ilvG p-YZdown, and a correct PCR product should be 2993 bp. A correct single colony is picked, and named as Sval0121.

In a second step, a genomic DNA of M1-93 is used as a template, and 188 bp of a DNA fragment II is amplified by using primers ilvG pro-up/ilvG pro-down. The DNA fragment II is used for the second homologous recombination. The DNA fragment II is electrotransformed into strain Sval021.

Electrotransformation conditions and steps are the same as the second step method for the *mgsA* gene knockout described in Example 1. Colonies were PCR verified using primers ilvG pro-YZup / ilvG p-YZ down and sequenced, and a correct colony amplification product is 462 bp. A correct single colony is picked, and named as Sval022.

### Example 12: Mutation of acetolactate synthase gene ilvH

A mutation is transferred into the *ilvH* gene so as to release feedback inhibition of L-valine through a two-step homologous recombination method. Specific steps are as follows.

In a first step, a pXZ-CS plasmid DNA is used as a template, 2719 bp of a DNA fragment I is amplified by using primers ilvH*-cat-up/ ilvH*-cat-down, and used for the first step of the homologous recombination. Amplification system and amplification conditions are the same as those described in Example 1.

The DNA fragment I is used for the first homologous recombination: firstly, a pKD46 plasmid is transformed into *Escherichia coli* Sval022 by an electrotransformation method, and then the DNA fragment I is electrotransformed into the *Escherichia coli* Sval022 with the pKD46.

Electrotransformation conditions and steps are the same as the first step method for the *mgsA* gene knockout described in Example 1. 200 µl of bacterial solution is spreaded onto a LB plate containing ampicillin (a final concentration is 100 µg/ml) and chloramphenicol (a final concentration is 34 µg/ml). After being cultured overnight at 30°C, colonies were PCR verified using primers ilvH*-mutYZ-up/ ilvH*-mut-down, and a correct PCR product should be 3165 bp. A correct single colony is picked, and named as Sval023.

In a second step, a DNA of wild-type *Escherichia coli ATCC* 8739 is used as a template, and 467 bp of a DNA fragment II is amplified by using primers ilvH*-mut-up / ilvH*-mut-down. The DNA fragment II is used for the second homologous recombination. The DNA fragment II is electrotransformed into strain Sval023.

Electrotransformation conditions and steps are the same as the second step method for the *mgsA* gene knockout described in Example 1. Colonies were PCR verified using primers ilvH*-mutYZ-up/ ilvH*-mut-down, and a correct colony amplification product is 619 bp. A correct single colony is picked, and named as Sval024.

### Example 13: Fermentation and production of L-valine using recombinant strain Sval024

A seed culture medium is formed by the following components (a solvent is water):
Glucose 20 g/L, corn syrup dry powder 10 g/L, KH₂PO₄ 8.8 g/L, (NH₄)₂SO₄ 2.5 g/L, and MgSO₄ •7H₂O 2 g/L.

The fermentation culture medium is most the same as the seed culture medium, and a difference is only that the glucose concentration is 50 g/L.

Anaerobic fermentation of Sval024 includes the following steps:
(1) Seed culture: a fresh clone on an LB plate is inoculated into a test tube containing 4 ml of the seed culture medium, and shake-cultured overnight at 37°C and 250 rpm. Then, a culture is transferred to 250 ml of a triangular flask containing 30 ml of the seed culture medium according to an inoculum size of 2% (V/V), and seed culture solution is obtained by shake culture at 37°C and 250 rpm for 12 hours, and used for fermentation medium inoculation.
(2) Fermentation culture: a volume of the fermentation culture medium in 500 ml of an fermenter is 250 ml, and the seed culture solution is inoculated into the fermentation culture medium according to an inoculum size of final concentration OD550=0.1, and fermented at 37°C and 150 rpm for 4 days, to obtain fermentation solution. The neutralizer is 5M ammonia, the pH was is controlled at 7.0. No air was sparged during the fermentation.

Analytical method: an Agilent (Agilent-1260) high performance liquid chromatograph is used to determine components in the fermentation solution after fermentation for 4 days. The concentrations of glucose and organic acid in the fermentation solution are determined by using an Aminex HPX-87H organic acid analytical column of Biorad Company. A Sielc amino acid analysis column primesep 100 250×4.6mm is used for amino acid determination.

It is discovered from results that: strain Sval024 could produce 1.3 g/L of L-valine (L-valine peak corresponding to a position in Fig. 2 appears) with a yield of 0.31 mol/mol after 4 days fermentation under anaerobic conditions.

### Example 14: Cloning and integration of leucine dehydrogenase encoding gene leuDH

Referring to the reported (Ohshima, T. et.al, Properties of crystalline leucine dehydrogenase from Bacillus sphaericus. The Journal of biological chemistry 253, 5719-5725 (1978)) sequence of a leuDH from Lysinibacillus sphaericus IFO 3525, a leuDH gene was codon optimized and chemically synthesized (an optimized sequence is as shown in a sequence number 90). During the synthesis, an M1-93 artificial regulatory element is added before the *leuDH* gene to initiate expression of the *leuDH* gene, and inserted into a pUC57 vector to construct a plasmid pUC57-M1-93-leuDH (gene synthesis and vector construction are completed by Nanjing Genscript Biotechnology Co., Ltd.). The M1-93 artificial regulatory element and the *leuDH* gene are integrated into the fumarate reductase encoding gene *frd* site in strain Sval024 through a two-step homologous recombination method and subtitute the *frd* gene, namely the *frd* gene is knocked out while the *leuDH* is integrated. Specific steps are as follows.

In a first step, a pXZ-CS plasmid DNA is used as a template, 2719 bp of a DNA fragment I is amplified by using primers frd-cs-up / frd-cs-down, and used for the first step of the homologous recombination. Amplification system and amplification conditions are the same as those described in Example 1.

The DNA fragment I is used for the first homologous recombination: firstly, a pKD46 plasmid is transformed into *Escherichia coli* Sval024 by an electrotransformation method, and then the DNA fragment I is electrotransformed into the *Escherichia coli* Sval024 with the pKD46.

Electrotransformation conditions and steps are the same as the first step method for the *mgsA* gene knockout described in Example 1. 200 µl of bacterial solution is spreaded onto a LB plate containing ampicillin (a final concentration is 100 µg/ml) and chloramphenicol (a final concentration is 34 µg/ml). After being cultured overnight at 30°C, colonies were PCR verified using primers XZ-frd-up/XZ-frd-down, and a correct PCR product should be 3493 bp. A correct single colony is picked, and named as Sval025.

In a second step, a pUC57-M1-93-leuDH plasmid DNA is used as a template, and 1283 bp of a DNA fragment II is amplified by using primers frd-M93-up/frd-leuDH-down. The DNA fragment II is used for the second homologous recombination. The DNA fragment II is electrotransformed into strain Sval025.

Electrotransformation conditions and steps are the same as the second step method for the *mgsA* gene knockout described in Example 1. Colonies were PCR verified using primers XZ-frd-up/XZ-frd-down, and a correct colony amplification product is 2057 bp. A correct single colony is picked, and named as Sval026.

### Example 15: Fermentation and production of L-valine using recombinant strain Sval026

Components and preparation of seed culture medium and fermentation culture medium are the same as those described in Example 13.

The fermentation is performed in 500 mL of a fermentation vessel, and a fermentation process and an analysis process are the same as the fermentation process and the analysis process of the Sval024 described in Example 13.

It is discovered from results that: the strain Sval026 could produce 1.8 g/L of L-valine (L-valine peak corresponding to a position in Fig. 2 appears) 0.56 mol/mol after 4 days fermentation under anaerobic conditions.

### Example 16: Regulation of 6-phosphate glucose dehydrogenase gene encoding zwf

Started from Sval026, an artificial regulatory element is used to regulate expression of *zwf* gene through a method of two-step homologous recombination, and recombinant *Escherichia coli* Sval041 are obtained. It specifically includes the following steps.

In a first step, a pXZ-CS plasmid DNA is used as a template, 2719 bp of a DNA fragment I is amplified by using primers Zwf-Pcat-up/ Zwf-PsacB-down, and used for the first step of the homologous recombination.

The DNA fragment I is used for the first homologous recombination: firstly, a pKD46 plasmid is transformed into *Escherichia coli* Sval026 by an electrotransformation method, and then the DNA fragment I is electrotransformed into the *Escherichia coli* Sval026 with the pKD46.

Electrotransformation conditions and steps are the same as the first step method for the *mgsA* gene knockout described in Example 1. 200 µl of bacterial solution is spreaded onto a LB plate containing ampicillin (a final concentration is 100 µg/ml) and chloramphenicol (a final concentration is 34 µg/ml). After being cultured overnight at 30°C, colonies were PCR verified using primers zwf-YZ442-up/ zwf-YZ383-down, and a correct colony amplification product is 3339 bp. A correct single colony is picked, and named as Sval041.

In a second step, a genomic DNA of M1-93 is used as a template, and 189 bp of a DNA fragment II is amplified by using primers zwf-RBS1-up/zwf-RBS1-down, and used for the second homologous recombination. Amplification conditions and system are the same as those described in (1). The DNA fragment II is electrotransformed into strain Sval041.

Electrotransformation conditions and steps are the same as the second step method for the *mgsA* gene knockout described in Example 1. Colonies were PCR verified using primers zwf-YZ442-up/ zwf-YZ383-down, and a correct colony amplification product is 809 bp. A correct single colony is picked, and named as Sval042 (Table 1).

### Example 17: Regulation of lactonase encoding gene pgl

Started from Sval033, an artificial regulatory element is used to regulate expression of a *pgl* gene through a method of two-step homologous recombination, and recombinant *Escherichia coli* Sval042 are acquired. It specifically includes the following steps.

In a first step, a pXZ-CS plasmid DNA is used as a template, 2719 bp of a DNA fragment I is amplified by using primers pgl-Pcat-up/ pgl-PsacB-down, and used for the first step of the homologous recombination.

The DNA fragment I is used for the first homologous recombination: firstly, a pKD46 plasmid is transformed into *Escherichia coli* Sval033 by an electrotransformation method, and then the DNA fragment I is electrotransformed into the *Escherichia coli* Sval042 with the pKD46.

Electrotransformation conditions and steps are the same as the first step method for the *mgsA* gene knockout described in Example 1. 200 µl of bacterial culture is spreaded onto a LB plate containing ampicillin (a final concentration is 100 µg/ml) and chloramphenicol (a final concentration is 34 µg/ml). After being cultured overnight at 30°C, colonies were PCR verified using primers pgl-YZ308-up/ pgl-YZ341-down, and a correct PCR product should be 3248 bp. A correct single colony is picked, and named as Sval043.

In a second step, a genomic DNA of M1-93 (Lu, et al., Appl Microbiol Biotechnol, 2012,93:2455-2462) is used as a template, and 189 bp of a DNA fragment II is amplified by using primers pgl-RBS2-up/pgl-RBS2-down, and used for the second homologous recombination. Amplification conditions and system are the same as those described in (1). The DNA fragment II is electrotransformed into strain Sval043.

Electrotransformation conditions and steps are the same as the second step method for the *mgsA* gene knockout described in Example 1. Colonies were PCR verified using primers pgl-YZ308-up/ pgl-YZ341-down, and a correct colony amplification product is 718 bp. A correct single colony is picked, and named as Sval044 (Table 1).

### Example 18: Cloning and integration of edd and eda genes from Zymomonas mobilis ZM4 strain

According to sequences of edd and *eda* genes derived from *Zymomonas mobilis* ZM4 reported by a literature (The genome sequence of the ethanologenic bacterium *Zymomonas mobilis* ZM4, Nat. Biotechnol., 2005, 23(1):63-68), the edd and *eda* genes derived from the *Zymomonas mobilis* ZM4 are synthesized by whole genes, herein an artificial regulatory element MRS1 is added before the *edd* gene, and the *edd* and *eda* genes are connected by an artificial RBS regulatory element (CAGGAAACAGCT). A whole-gene-synthesized MRS1-edd-RBS-eda fragment is linked to a pUC57 vector through EcoRV. The whole gene synthesis of the sequence is completed by Nanjing Genscript Biotechnology Co., Ltd., and the pUC57 vector is also from Nanjing Genscript Biotechnology Co., Ltd. The MRS1-edd-RBS-eda is integrated into an *edd-eda* site of the *Escherichia coli* itself through a two-step homologous recombination method and replaces the *edd-eda* gene of the *Escherichia coli* itself. Specific steps are as follows.

In a first step, a pXZ-CS plasmid (Tan, et al., Appl Environ Microbiol, 2013, 79:4838-4844) DNA is used as a template, 2719 bp of a DNA fragment I is amplified by using primers edd-cat-up/eda-sacB-down, and used for the first step of the homologous recombination.

The DNA fragment I is used for the first homologous recombination: firstly, a pKD46 plasmid is transformed into *Escherichia coli* Sval044 by an electrotransformation method, and then the DNA fragment I is electrotransformed into the *Escherichia coli* Sval044 with the pKD46.

Electrotransformation conditions and steps are the same as the first step method for the *mgsA* gene knockout described in Example 1. 200 µl of bacterial solution is spreaded onto a LB plate containing ampicillin (a final concentration is 100 µg/ml) and chloramphenicol (a final concentration is 34 µg/ml). After being cultured overnight at 30°C, colonies were PCR verified using primers edd-YZ-up/edd-YZ-down, and a correct PCR product should be 3123 bp. A correct single colony is picked, and named as Sval045.

In a second step, a plasmid DNA of pUC57-MRS1-edd-eda is used as a template, and 2651 bp of a DNA fragment II is amplified by using primers Edd-int-up/ Eda-int-down, and used for the second homologous recombination. Amplification conditions and system are the same as those described in (1). The DNA fragment II is electrotransformed into strain Sval045.

Electrotransformation conditions and steps are the same as the second step method for the *mgsA* gene knockout described in Example 1. Colonies were PCR verified using primers edd-YZ-up/edd-YZ-down, and a correct colony amplification product is 3055 bp . A correct single colony is picked, and named as Sval046 (Table 1).

### Example 19: Knockout of 6-phosphoglucokinase encoding gene pfkA

In an strain Sval037, a key enzyme 6-phosphoglucokinase gene *pfkA* in a glycolytic pathway is knocked out to achieve that a recombinant strain uses an ED pathway to ferment and generate L-valine. The *pfkA* gene is knocked out through a method of two-step homologous recombination, and specific steps are as follows.

In a first step, a pXZ-CS plasmid (Tan, et al., Appl Environ Microbiol, 2013, 79:4838-4844) DNA is used as a template, 2719 bp of a DNA fragment I is amplified by using primers pfkAdel-cat-up/pfkAdel-sacB-down, and used for the first step of the homologous recombination.

The DNA fragment I is used for the first homologous recombination: firstly, a pKD46 plasmid (Datsenko and Wanner 2000, Proc Natl Acad Sci USA 97:6640-6645; and the plasmid is purchased from Coil Genetic Stock Center (CGSC) of Yale University, CGSC#7739) is transformed into *Escherichia coli* Sval046 by an electrotransformation method, and then the DNA fragment I is electrotransformed into the *Escherichia coli* Sval046 with the pKD46.

Electrotransformation conditions and steps are the same as the first step method for the *mgsA* gene knockout described in Example 1. 200 µl of bacterial solution is spreaded onto a LB plate containing ampicillin (a final concentration is 100 µg/ml) and chloramphenicol (a final concentration is 34 µg/ml). After being cultured overnight at 30°C, colonies were PCR verified using primers pfkAdel-up/pfkAdel-YZ-down, and a correct PCR product should be 3145 bp. A correct single colony is picked, and named as Sval047.

In a second step, a genomic DNA of wild-type ATCC8739 is used as a template, and 379 bp of a DNA fragment II is amplified by using primers pfkAdel-up / pfkAdel-down, and used for the second homologous recombination. Amplification conditions and system are the same as those described in (1). The DNA fragment II is electrotransformed into strain Sval047.

Electrotransformation conditions and steps are the same as the second step method for the *mgsA* gene knockout described in Example 1. Colonies were PCR verified using primers pfkAdel-up/pfkAdel-YZ-down, and a correct colony amplification product is 526 bp. A correct single colony is picked, and named as Sval048 (Table 1).

### Example 20: production of L-valine using recombinant strain Sval048

Components and preparation of seed culture medium and fermentation culture medium are the same as those described in Example 13.

The fermentation is performed in 500 mL of a fermentation vessel, a fermentation process and an analysis process are the same as the fermentation process and the analysis process of the Sval024 described in Example 13.

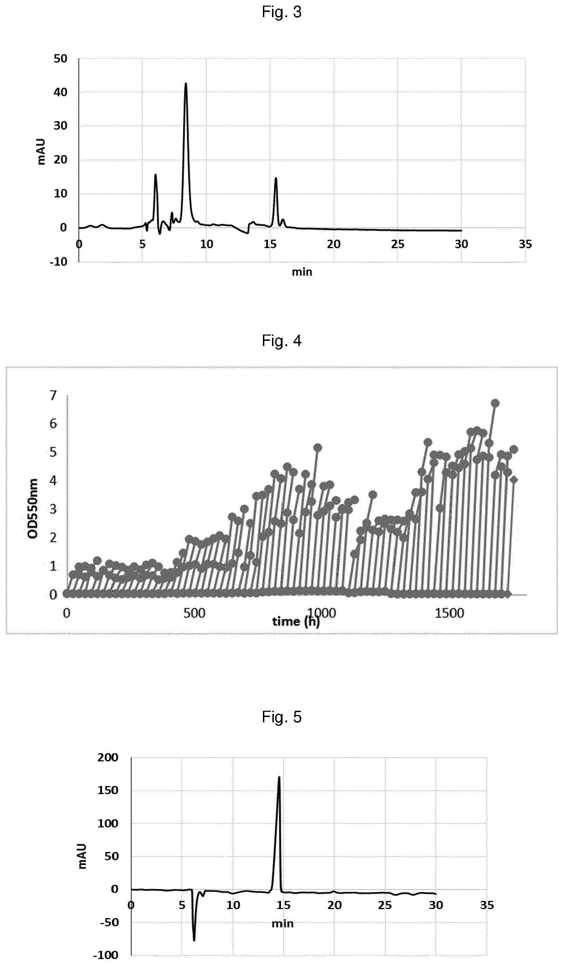

It is discovered from results that: the strain Sval048 could produce 1.9 g/L of L-valine with a yield of 0.82 mol/mol after fermentation for 4 days under anaerobic conditions. Fig. 2 is a spectrum of L-valine standard substance, and Fig. 3 is a spectrum of Sval048 fermentation solution.

### Example 21: Construction of recombinant strain Sval049

Started from Sval048, cell growth and L-valine production capacity are synchronously improved through metabolic evolution.

Metabolic evolution was carried out in 500 ml fermentation vessel with 250 ml fermentation culture medium. The fermentative pH was controlled at 7.0 by using 5 Mammonia as neutralizer. Components and preparation of the fermentation culture medium used for the metabolic evolution are the same as those of the fermentation culture medium described in Example 18. Every 24 hours, fermentation solution is transferred into a new fermentation vessel, and the initial OD550 is 0.1. After 100 generations of the evolution, a strain Sval049 is obtained (Fig. 4). The strain Sval049 is preserved in China General Microbiological Culture Collection Center (CGMCC) with a deposit number CGMCC 19457.

### Example 22: Fermentation of strain Sval049 to produce L-valine in 500 mL fermentation vessel

Components and preparation of a seed culture medium are the same as those described in Example 13.

The fermentation is performed in 500 mL of a fermentation vessel, and a fermentation culture medium is 250 ml. The fermentation culture medium is basically the same as the seed culture medium. A difference is that a glucose concentration is 100 g/L, and a neutralizer used is 5M ammonia, so that fermentative pH is controlled in 7.0.

It is discovered from results that: after fermented in 500 mL of the fermentation vessel for 48 hours, strain Sval049 produced 47 g/L with a yield of 0.91 mol/mol, and impurities such as a heteroacid are not generated.

### Example 23: Production of L-valine by fermentation of recombinant strain Sval049 in 5 L fermentation vessel

Components, preparation and analytical method of a seed culture medium are the same as those described in Example 13. A fermentation culture medium is basically the same as the seed culture medium, and a difference is that a glucose concentration is 140 g/L.

The fermentation is performed in 5 L of a fermentation vessel (Shanghai Baoxing, BIOTECH-5BG) under anaerobic conditions, including the following steps:
(1) Seed culture: the seed culture medium in 500 ml of a triangular flask is 150 ml, and it is sterilized at 115°C for 15 min. After cooling, recombinant *Escherichia coli* Sval041 are inoculated into the seed culture medium according to an inoculum size of 1% (V/V), and cultured at 37°C and 100 rpm for 12 hours to obtain seed solution for inoculation of the fermentation culture medium.
(2) Fermentation culture: a volume of the fermentation culture medium in 5 L is 3 L, and it is sterilized at 115°C for 25 min. The seed solution is inoculated into the fermentation culture medium according to an inoculum size of final concentration OD550=0.2, and cultured under anaerobic conditions at 37°C for 48 hours, and a stirring speed is 200 rpm, fermentation solution is obtained. The fermentation solution is all of substances in the fermentation vessel. No air was sparged during the fermentation.

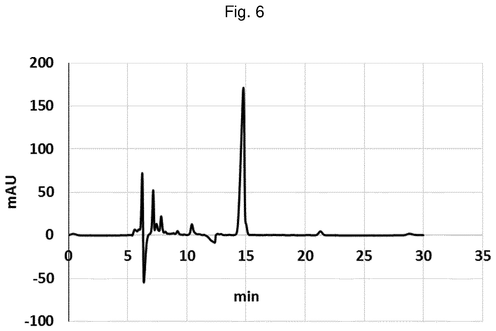

It is discovered from results that: after fermented in 5 L of the fermentation vessel for 48 hours, Sval049 produced 87 g/L L-valine with a yield of 0.92 mol/mol, and impurities such as a heteroacid are not generated. Fig. 5 is a spectrum of L-valine standard substance, and Fig. 6 is a spectrum of Sval049 fermentation solution.

## Claims

1. A construction method of a recombinant microorganism for producing L-valine comprising:
transferring an amino acid dehydrogenase gene into a microorganism and/or activating an Entner-Doudoroff metabolic pathway in the microorganism;
optionally, further comprising a step of knocking out a 6-phosphoglucokinase gene *pfkA;*
optionally, further comprising a step of transferring an acetohydroxy acid reductoisomerase encoding gene into the microorganism; the acetohydroxy acid reductoisomerase encoding gene is preferably *ilvC;*
preferably, the amino acid dehydrogenase gene is NADH-dependent;
preferably, the amino acid dehydrogenase gene is a leucine dehydrogenase gene; and
preferably, the activation of the Entner-Doudoroff metabolic pathway comprises a step of improving expression intensity of a *zwf* gene, a *pgl* gene, an *edd* gene and an *eda* gene.

2. The construction method according to claim 1, wherein the method further comprises one or more of the following modifications (1)-(7) to the recombinant microorganism according to claim 1:
(1) knocking out a gene *mgsA;*
(2) knocking out a gene *ldhA;*
(3) knocking out genes *pta* and/or *ackA;*
(4) knocking out genes *tdcD* and/or *tdcE;*
(5) knocking out a gene *adhE;*
(6) knocking out genes *frd* and/or *pflB;* and
(7) enhancing activity of AHAS and/or *ilvD;*
preferably, the AHAS is ilvBN, or ilvGM, or ilvlH; optionally, the activity of the ilvlH is enhanced by releasing feedback inhibition of valine to the ilvH, preferably, the ilvH gene enhanced by mutation;
preferably, the above item (7) is selected for modification;
preferably, the above items (7) and (2) are selected for modification;
preferably, the above items (7) and (6) are selected for modification;
preferably, the above items (7), (2) and (5) are selected for modification;
preferably, the above items (7), (2) and (6) are selected for modification;
preferably, the above items (7), (1), and (3)-(6) are selected for modification;
preferably, the above items (1)-(7) are selected for modification;
preferably, the item (6) is achieved by substituting the *pflB* gene of the microorganism itself with the *ilvD* gene;
preferably, the item (6) is achieved by substituting the *frd* gene of the microorganism itself with the leuDH gene; and
preferably, the item (1) is achieved by substituting the *mgsA* gene of the microorganism itself with the *ilvC* gene.

3. The construction method according to claim 1 or 2, wherein the microorganism is *Escherichia coli;* and more preferably, the microorganism is *Escherichia coli ATCC* 8739.

4. The construction method according to any one of claims 1 to 3, wherein at least one regulatory element is used to activate or enhance activity of encoding genes of *ilvD, leuDH, ilvBN, zwf, pgl, ilvGM, edd, eda* or *ilvC;*
preferably, the regulatory element is selected from an M1-93 artificial regulatory element, an MRS1 artificial regulatory element, a RBS artificial regulatory element or an M1-46 artificial regulatory element;
preferably, the M1-93 artificial regulatory element regulates *ilvD, leuDH, ilvBN, zwf, pgl* and *ilvGM* genes;
the MRS1 artificial regulatory element regulates the *edd* gene;
the RBS artificial regulatory element regulates the gene *eda;* and
the M1 -46 artificial regulatory element regulates the *ilvc* gene.

5. The construction method according to any one of claims 1-4, wherein one or more copies of the enzyme encoding gene and the regulatory element are integrated into a genome of the microorganism, or a plasmid containing the enzyme encoding gene is transferred into the microorganism;
preferably, transfer, mutation, knockout, activation or regulation of the enzyme gene is completed by a method of integrating into the genome of the microorganism;
preferably, the transfer, mutation, knockout, activation or regulation of the enzyme gene is completed by a homologous recombination method; and
preferably, the transfer, mutation, knockout, activation or regulation of the enzyme gene is completed by a two-step homologous recombination method.

6. A recombinant microorganism obtained by the construction method according to any one of claims 1-5.

7. A method for acquiring a recombinant microorganism for highly producing L-valine, wherein it is obtained through metabolic evolution on the basis of the recombinant microorganism according to claim 6.

8. A recombinant microorganism, wherein a deposit number thereof is CGMCC 19457.

9. An application of the recombinant microorganism according to claim 6 or claim 8 in production of L-valine.

10. A method for producing L-valine, wherein the method comprises: (1) fermenting and culturing the recombinant microorganism according to claim 6 or 8; and (2) separating and harvesting L-valine; preferably, the fermentation is carried out under anaerobic conditions.


SEQUENCE LISTING

<110>  Bayannnaoer Huaheng Biological Engineering Co., Ltd.
       Tianjin Institute of Industrial Biotechnology, Chinese Academy of
Sciences

<120>  Recombinant microorganism for producing L-valine, construction method
and application thereof

<130>  PN151380HHSW

<150>  CN202010460035.9
<151>  2020-05-27

<160>  90

<170>  PatentIn version 3.5

<210>  1
<211>  73
<212>  DNA
<213>  Escherichia coli

<400>  1
gtaggaaagt taactacgga tgtacattat ggaactgacg actcgcactt tgtgacggaa      60

gatcacttcg cag                                                         73


<210>  2
<211>  75
<212>  DNA
<213>  Escherichia coli

<400>  2
gcgtttgcca cctgtgcaat attacttcag acggtccgcg agataacgct ttatttgtta      60

actgttaatt gtcct                                                       75


<210>  3
<211>  20
<212>  DNA
<213>  Escherichia coli

<400>  3
cagctcatca accaggtcaa                                                  20


<210>  4
<211>  20
<212>  DNA
<213>  Escherichia coli

<400>  4
aaaagccgtc acgttattgg                                                  20


<210>  5
<211>  70
<212>  DNA
<213>  Escherichia coli

<400>  5
gcgtttgcca cctgtgcaat attacttcag acggtccgcg agataacgct aagtgcgagt      60

cgtcagttcc                                                              70


<210>  6
<211>  72
<212>  DNA
<213>  Escherichia coli

<400>  6
gtaggaaagt taactacgga tgtacattat ggaactgacg actcgcactt atggctaact      60

acttcaatac ac                                                           72


<210>  7
<211>  75
<212>  DNA
<213>  Escherichia coli

<400>  7
gcgtttgcca cctgtgcaat attacttcag acggtccgcg agataacgct ttaacccgca      60

acagcaatac gtttc                                                        75


<210>  8
<211>  73
<212>  DNA
<213>  Escherichia coli

<400>  8
gtaggaaagt taactacgga tgtacattat ggaactgacg actcgcactt tgtgacggaa      60

gatcacttcg cag                                                          73


<210>  9
<211>  75
<212>  DNA
<213>  Escherichia coli

<400>  9
agctgtgcca gctgctggcg cagattcagt gtattgaagt agttagccat ttatttgtta      60

actgttaatt gtcct                                                        75


<210>  10
<211>  70
<212>  DNA
<213>  Escherichia coli

<400>  10
gtaggaaagt taactacgga tgtacattat ggaactgacg actcgcactt ttatctctgg      60

cggtgttgac                                                              70


<210>  11
<211>  72

<212>  DNA
<213>  Escherichia coli

<400>  11
agctgtgcca gctgctggcg cagattcagt gtattgaagt agttagccat agctgtttcc      60

tggtttaaac cg                                                          72


<210>  12
<211>  20
<212>  DNA
<213>  Escherichia coli

<400>  12
cgcactacat cagagtgctg                                                  20


<210>  13
<211>  73
<212>  DNA
<213>  Escherichia coli

<400>  13
ttcaacatca ctggagaaag tcttatgaaa ctcgccgttt atagcacaaa tgtgacggaa      60

gatcacttcg cag                                                         73


<210>  14
<211>  75
<212>  DNA
<213>  Escherichia coli

<400>  14
agcggcaaga ttaaaccagt tcgttcgggc aggtttcgcc tttttccaga ttatttgtta      60

actgttaatt gtcct                                                       75


<210>  15
<211>  20
<212>  DNA
<213>  Escherichia coli

<400>  15
gataacggag atcgggaatg                                                  20


<210>  16
<211>  20
<212>  DNA
<213>  Escherichia coli

<400>  16
ctttggctgt cagttcacca                                                  20


<210>  17
<211>  71
<212>  DNA
<213>  Escherichia coli

<400>  17
agcggcaaga ttaaaccagt tcgttcgggc aggtttcgcc tttttccaga tttgtgctat      60

aaacggcgag t                                                           71


<210>  18
<211>  73
<212>  DNA
<213>  Escherichia coli

<400>  18
aggtacttcc atgtcgagta agttagtact ggttctgaac tgcggtagtt tgtgacggaa      60

gatcacttcg cag                                                         73


<210>  19
<211>  75
<212>  DNA
<213>  Escherichia coli

<400>  19
ggtcggcaga acgctgtacc gctttgtagg tggtgttacc ggtgttcaga ttatttgtta      60

actgttaatt gtcct                                                       75


<210>  20
<211>  20
<212>  DNA
<213>  Escherichia coli

<400>  20
cgggacaacg ttcaaaacat                                                  20


<210>  21
<211>  20
<212>  DNA
<213>  Escherichia coli

<400>  21
attgcccatc ttcttgttgg                                                  20


<210>  22
<211>  71
<212>  DNA
<213>  Escherichia coli

<400>  22
ggtcggcaga acgctgtacc gctttgtagg tggtgttacc ggtgttcaga aactaccgca      60

gttcagaacc a                                                           71


<210>  23
<211>  73
<212>  DNA
<213>  Escherichia coli

<400>  23

ccgtgattgg tctgctgacc atcctgaaca tcgtatacaa actgttttaa tgtgacggaa 60

gatcacttcg cag 73

<210> 24
<211> 75
<212> DNA
<213> Escherichia coli

<400> 24
cgcctggggc acgttgcgtt tcgataatct ttttcataca tcctccggcg ttatttgtta 60

actgttaatt gtcct 75

<210> 25
<211> 21
<212> DNA
<213> Escherichia coli

<400> 25
tgatgagcta cctggtatgg c 21

<210> 26
<211> 24
<212> DNA
<213> Escherichia coli

<400> 26
cgccgacaga gtaataggtt ttac 24

<210> 27
<211> 77
<212> DNA
<213> Escherichia coli

<400> 27
cgcctggggc acgttgcgtt tcgataatct ttttcataca tcctccggcg ttaaaacagt 60

ttgtatacga tgttcag 77

<210> 28
<211> 72
<212> DNA
<213> Escherichia coli

<400> 28
ataactctaa tgtttaaact cttttagtaa atcacagtga gtgtgagcgc tgtgacggaa 60

gatcacttcg ca 72

<210> 29
<211> 75
<212> DNA
<213> Escherichia coli

<400> 29
ccgtttatgt tgccagacag cgctactgat taagcggatt ttttcgcttt ttatttgtta 60

actgttaatt gtcct                                                    75


<210>  30
<211>  74
<212>  DNA
<213>  Escherichia coli

<400>  30
ccgtttatgt tgccagacag cgctactgat taagcggatt ttttcgcttt gcgctcacac      60

tcactgtgat ttac                                                     74


<210>  31
<211>  21
<212>  DNA
<213>  Escherichia coli

<400>  31
catgctaatg tagccaccaa a                                             21


<210>  32
<211>  20
<212>  DNA
<213>  Escherichia coli

<400>  32
ttgcaccacc atccagataa                                               20


<210>  33
<211>  73
<212>  DNA
<213>  Escherichia coli

<400>  33
aaacgaccac cattaatggt tgtcgaagta cgcagtaaat aaaaaatcca tgtgacggaa      60

gatcacttcg cag                                                      73


<210>  34
<211>  75
<212>  DNA
<213>  Escherichia coli

<400>  34
cggtccgaac ggcgcgccag cacgacgacc gtctggggtg ttacccgttt ttatttgtta      60

actgttaatt gtcct                                                    75


<210>  35
<211>  70
<212>  DNA
<213>  Escherichia coli

<400>  35
aaacgaccac cattaatggt tgtcgaagta cgcagtaaat aaaaaatcca atgcctaagt      60

accgttccgc                                                              70

<210>  36
<211>  74
<212>  DNA
<213>  Escherichia coli

<400>  36
cggtccgaac ggcgcgccag cacgacgacc gtctggggtg ttacccgttt ttaacccccc      60

agtttcgatt tatc                                                         74

<210>  37
<211>  21
<212>  DNA
<213>  Escherichia coli

<400>  37
ctgcggagcc gatctcttta c                                                 21

<210>  38
<211>  22
<212>  DNA
<213>  Escherichia coli

<400>  38
cgagtaataa cgtcctgctg ct                                                22

<210>  39
<211>  72
<212>  DNA
<213>  Escherichia coli

<400>  39
aaacgaccac cattaatggt tgtcgaagta cgcagtaaat aaaaaatcca tgtgacggaa      60

gatcacttcg ca                                                           72

<210>  40
<211>  75
<212>  DNA
<213>  Escherichia coli

<400>  40
cccgccatat tacgaccatg agtggtggtg gcggaacggt acttaggcat ttatttgtta      60

actgttaatt gtcct                                                        75

<210>  41
<211>  70
<212>  DNA
<213>  Escherichia coli

<400>  41
aaacgaccac cattaatggt tgtcgaagta cgcagtaaat aaaaaatcca ttatctctgg      60

cggtgttgac                                                              70

<210>  42
<211>  78
<212>  DNA
<213>  Escherichia coli

<400>  42
cccgccatat tacgaccatg agtggtggtg gcggaacggt acttaggcat tgctgacctc      60

ctggtttaaa cgtacatg                                                    78


<210>  43
<211>  20
<212>  DNA
<213>  Escherichia coli

<400>  43
caaccagatc gagcttgatg                                                  20


<210>  44
<211>  20
<212>  DNA
<213>  Escherichia coli

<400>  44
tgcagaaaac catcgacaag                                                  20


<210>  45
<211>  20
<212>  DNA
<213>  Escherichia coli

<400>  45
caccaatcag cgtgacaact                                                  20


<210>  46
<211>  72
<212>  DNA
<213>  Escherichia coli

<400>  46
gaaggcgaat ggctgagatg aaaaacctga aaattgaggt ggtgcgctat tgtgacggaa      60

gatcacttcg ca                                                          72


<210>  47
<211>  75
<212>  DNA
<213>  Escherichia coli

<400>  47
tctcaggctc cttaccagta cagggcaaca aacaggatta cgatggtggc ttatttgtta      60

actgttaatt gtcct                                                       75


<210>  48

<211>  70
<212>  DNA
<213>  Escherichia coli

<400>  48
gaaggcgaat ggctgagatg aaaaacctga aaattgaggt ggtgcgctat ttatctctgg      60

cggtgttgac                                                              70


<210>  49
<211>  77
<212>  DNA
<213>  Escherichia coli

<400>  49
tctcaggctc cttaccagta cagggcaaca aacaggatta cgatggtggc ttaacggccg      60

ttcaaaatat tttttc                                                       77


<210>  50
<211>  72
<212>  DNA
<213>  Escherichia coli

<400>  50
ctgacgaaac ctcgctccgg cggggttttt tgttatctgc aattcagtac tgtgacggaa      60

gatcacttcg ca                                                           72


<210>  51
<211>  75
<212>  DNA
<213>  Escherichia coli

<400>  51
tctgcgccgg taaagcgctt acgcgtcgat gttgtgcccg aacttgccat ttatttgtta      60

actgttaatt gtcct                                                        75


<210>  52
<211>  70
<212>  DNA
<213>  Escherichia coli

<400>  52
ctgacgaaac ctcgctccgg cggggttttt tgttatctgc aattcagtac ttatctctgg      60

cggtgttgac                                                              70


<210>  53
<211>  70
<212>  DNA
<213>  Escherichia coli

<400>  53
tctgcgccgg taaagcgctt acgcgtcgat gttgtgcccg aacttgccat agctgtttcc      60

tggtttaaac                                                              70

<210>  54
<211>  22
<212>  DNA
<213>  Escherichia coli

<400>  54
gttctgcgcg gaacacgtat ac                                                22


<210>  55
<211>  20
<212>  DNA
<213>  Escherichia coli

<400>  55
ccgctacagg ccatacagac                                                   20


<210>  56
<211>  72
<212>  DNA
<213>  Escherichia coli

<400>  56
tgaactaaga ggaagggaac aacattcaga ccgaaattga atttttttca tgtgacggaa      60

gatcacttcg ca                                                           72


<210>  57
<211>  75
<212>  DNA
<213>  Escherichia coli

<400>  57
ttcacaccct gtgcccgcaa cgcatgtacc acccactgtg cgccattcat ttatttgtta      60

actgttaatt gtcct                                                        75


<210>  58
<211>  70
<212>  DNA
<213>  Escherichia coli

<400>  58
tgaactaaga ggaagggaac aacattcaga ccgaaattga atttttttca ttatctctgg      60

cggtgttgac                                                              70


<210>  59
<211>  71
<212>  DNA
<213>  Escherichia coli

<400>  59
ttcacaccct gtgcccgcaa cgcatgtacc acccactgtg cgccattcat agctgtttcc      60

tggtttaaac g                                                            71

<210>  60
<211>  22
<212>  DNA
<213>  Escherichia coli

<400>  60
gcataagata tcgctgctgt ag                                                22


<210>  61
<211>  20
<212>  DNA
<213>  Escherichia coli

<400>  61
gccagttttg ccagtagcac                                                   20


<210>  62
<211>  72
<212>  DNA
<213>  Escherichia coli

<400>  62
agaacctgat tatgcgccgg atattatcag tcttactcga aaatgaatca tgtgacggaa       60

gatcacttcg ca                                                           72


<210>  63
<211>  75
<212>  DNA
<213>  Escherichia coli

<400>  63
ttcatcgccc acggtctgga tggtcatacg cgataatgtc ggatcgtcgg ttatttgtta       60

actgttaatt gtcct                                                        75


<210>  64
<211>  74
<212>  DNA
<213>  Escherichia coli

<400>  64
agaacctgat tatgcgccgg atattatcag tcttactcga aaatgaatca gacgcgttat       60

tccgcgtgat tggc                                                         74


<210>  65
<211>  21
<212>  DNA
<213>  Escherichia coli

<400>  65
cacaccagag cgagcaacct c                                                 21


<210>  66
<211>  23

<212>  DNA
<213>  Escherichia coli

<400>  66
atgagctgga aagcaaactt agc                                              23


<210>  67
<211>  72
<212>  DNA
<213>  Escherichia coli

<400>  67
agttttgccg cactttgcgc gcttttcccg taatcgcacg ggtggataag tgtgacggaa      60

gatcacttcg ca                                                          72


<210>  68
<211>  75
<212>  DNA
<213>  Escherichia coli

<400>  68
gcgccgaaaa tgaccaggtc acaggcctgg gctgtttgcg ttaccgccat ttatttgtta      60

actgttaatt gtcct                                                       75


<210>  69
<211>  70
<212>  DNA
<213>  Escherichia coli

<400>  69
agttttgccg cactttgcgc gcttttcccg taatcgcacg ggtggataag ttatctctgg      60

cggtgttgac                                                             70


<210>  70
<211>  78
<212>  DNA
<213>  Escherichia coli

<400>  70
gcgccgaaaa tgaccaggtc acaggcctgg gctgtttgcg ttaccgccat attgtttctc      60

ctggtttaaa cgtacgtg                                                    78


<210>  71
<211>  20
<212>  DNA
<213>  Escherichia coli

<400>  71
cgaatggatc gcgttatcgg                                                  20


<210>  72
<211>  21
<212>  DNA

<213>  Escherichia coli

<400>  72
caaattgcgc caaaagtgct g                                                21


<210>  73
<211>  72
<212>  DNA
<213>  Escherichia coli

<400>  73
ttcagcattc accgccaaaa gcgactaatt ttagctgtta cagtcagttg tgtgacggaa      60

gatcacttcg ca                                                          72


<210>  74
<211>  75
<212>  DNA
<213>  Escherichia coli

<400>  74
acgtgaattt gctggctctc agggctggcg atataaactg tttgcttcat ttatttgtta      60

actgttaatt gtcct                                                       75


<210>  75
<211>  70
<212>  DNA
<213>  Escherichia coli

<400>  75
ttcagcattc accgccaaaa gcgactaatt ttagctgtta cagtcagttg ttatctctgg      60

cggtgttgac                                                             70


<210>  76
<211>  88
<212>  DNA
<213>  Escherichia coli

<400>  76
acgtgaattt gctggctctc agggctggcg atataaactg tttgcttcat acgtttcctc      60

ctggtttaaa cgtacatgct aacaatac                                         88


<210>  77
<211>  20
<212>  DNA
<213>  Escherichia coli

<400>  77
gatgaatagc gacgtgatgg                                                  20


<210>  78
<211>  20
<212>  DNA
<213>  Escherichia coli

<400>  78
ccatcttcca gacgcgttac                                                    20


<210>  79
<211>  72
<212>  DNA
<213>  Escherichia coli

<400>  79
tggtcgttcc tggaatgagt ttgagtaata tctgcgctta tcctttatgg tgtgacggaa        60

gatcacttcg ca                                                            72


<210>  80
<211>  75
<212>  DNA
<213>  Escherichia coli

<400>  80
gcaaaaaaac gctacaaaaa tgcccgatcc tcgatcgggc attttgactt ttatttgtta        60

actgttaatt gtcct                                                         75


<210>  81
<211>  70
<212>  DNA
<213>  Escherichia coli

<400>  81
tggtcgttcc tggaatgagt ttgagtaata tctgcgctta tcctttatgg ttatctctgg        60

cggtgttgac                                                               70


<210>  82
<211>  72
<212>  DNA
<213>  Escherichia coli

<400>  82
gcaaaaaaac gctacaaaaa tgcccgatcc tcgatcgggc attttgactt ttaggcaaca        60

gcagcgcgct tg                                                            72


<210>  83
<211>  20
<212>  DNA
<213>  Escherichia coli

<400>  83
gcatctggcg gatgcctatg                                                    20


<210>  84
<211>  23
<212>  DNA
<213>  Escherichia coli

<400>  84
caactgacca gtcagaatgt cac                                              23


<210>  85
<211>  72
<212>  DNA
<213>  Escherichia coli

<400>  85
ggtatcgacg cgctggtggt tatcggcggt gacggttcct acatgggtgc tgtgacggaa      60

gatcacttcg ca                                                          72


<210>  86
<211>  75
<212>  DNA
<213>  Escherichia coli

<400>  86
gtggcccagc acagttgcgc gggtttcacg accggtttct ttctcgatga ttatttgtta      60

actgttaatt gtcct                                                       75


<210>  87
<211>  21
<212>  DNA
<213>  Escherichia coli

<400>  87
atgattaaga aaatcggtgt g                                                21


<210>  88
<211>  70
<212>  DNA
<213>  Escherichia coli

<400>  88
gtggcccagc acagttgcgc gggtttcacg accggtttct ttctcgatga gcacccatgt      60

aggaaccgtc                                                             70


<210>  89
<211>  21
<212>  DNA
<213>  Escherichia coli

<400>  89
gtcgatgatg tcgtggtgaa c                                                21


<210>  90
<211>  1095
<212>  DNA
<213>  Lysinibacillus sphaericus

<400>  90
atggaaatct tcaagtatat ggaaaagtat gattatgaac aattggtatt ttgccaagac      60

gaagcatctg ggttaaaagc gattatcgct atccatgaca caacacttgg accagcatta 120

ggtggtgctc gtatgtggac ctacgcgaca gaagaaaatg cgattgagga tgcattaaga 180

ttagcacgcg ggatgacata taaaaatgca gctgctggtt taaaccttgg cggtggaaaa 240

acggtcatta ttggggaccc atttaaagat aaaaacgaag aaatgttccg tgctttaggt 300

cgtttcattc aaggattaaa cggtcgctat attaccgctg aagatgttgg tacaaccgta 360

acagatatgg atttaatcca tgaggaaaca aattacgtta caggtatatc gccagcgttt 420

ggttcatcgg gtaatccttc accagtaact gcttatggcg tttatcgtgg catgaaagca 480

gcggcgaaag aagcatttgg tacggatatg ctagaaggtc gtactatatc ggtacaaggg 540

ctaggaaacg tagcttacaa gctttgcgag tatttacata atgaaggtgc aaaacttgta 600

gtaacagata ttaaccaagc ggctattgat cgtgttgtca atgatttggg cgctacagca 660

gttgcacctg atgaaatcta ttcacaagaa gtcgatattt tctcaccgtg tgcacttggc 720

gcaattttaa atgacgaaac gattccgcaa ttaaaagcaa aagttattgc tggttctgct 780

aataaccaac tacaagattc acgacatgga gattatttac acgagctagg cattgtttat 840

gcacctgact atgtcattaa tgcaggtggt gtaataaatg tcgcggacga attatatggc 900

tataatcgtg aacgagcgtt gaaacgtgta gatggtattt acgatagtat tgaaaaaatc 960

tttgaaattt ccaaacgtga tagtattcca acatatgttg cggcaaatcg tttggcagaa 1020

gaacgtattg ctcgtgtagc gaaatcgcgt agtcagttct taaaaaatga aaaaaatatt 1080

ttgaacggcc gttaa 1095